(19) Europäisches Patentamt
European Patent Office
Office européen des brevets

(11) Veröffentlichungsnummer: **0 382 077 B1**

# (12) EUROPÄISCHE PATENTSCHRIFT

(45) Veröffentlichungstag der Patentschrift: **17.05.95**

(21) Anmeldenummer: **90101947.1**

(22) Anmeldetag: **01.02.90**

(51) Int. Cl.$^6$: **C07D 307/36**, C07D 333/08, C07D 207/333, C07D 231/12, C07D 261/08, C07D 233/64, C07D 271/06, C07D 237/08, A61K 31/34, A61K 31/38, A61K 31/40

## (54) Diarylsubstituierte heterocyclische Verbindung, ihre Herstellung und Arzneimittel und Kosmetika daraus.

(30) Priorität: **10.02.89 DE 3903993**

(43) Veröffentlichungstag der Anmeldung:
**16.08.90 Patentblatt 90/33**

(45) Bekanntmachung des Hinweises auf die Patenterteilung:
**17.05.95 Patentblatt 95/20**

(84) Benannte Vertragsstaaten:
**AT BE CH DE FR GB IT LI NL SE**

(56) Entgegenhaltungen:
**EP-A- 0 064 385**
**EP-A- 0 176 032**
**EP-A- 0 176 034**
**EP-A- 0 253 302**
**EP-A- 0 284 261**
**DE-A- 2 854 354**
**DE-A- 3 202 118**

(73) Patentinhaber: **BASF Aktiengesellschaft**
**Carl-Bosch-Strasse 38**
**D-67063 Ludwigshafen (DE)**

(72) Erfinder: **Wüst, Hans-Heiner, Dr.**
**Unteres Bieth 10**
**D-6915 Dossenheim (DE)**
Erfinder: **Janssen, Bernd, Dr.**
**Leuschnerstrasse 18 a**
**D-6700 Ludwigshafen (DE)**
Erfinder: **Murray, William V.**
**Township Line Road**
**Belle Mead, New Jersey 08502 (US)**
Erfinder: **Wachter, Michael P.**
**52 North Street**
**Bloomsbury, New Jersey 08804 (US)**
Erfinder: **Bell, Stanley**
**732 Braeburn Lane**
**Narbeth, Pennsylvania 19072 (US)**

EP 0 382 077 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue diarylsubstituierte fünf- oder sechsgliedrige heterocyclische Verbindungen, Verfahren zu deren Herstellung sowie deren Verwendung bei der Prophylaxe und Bekämpfung von Krankheiten.

Aus den DE-OS 2 854 354 und DE-OS 3 202 118 ist bekannt, daß retinoidale Benzoesäurederivate pharmakologische Wirkungen bei der topischen und systemischen Therapie von Neoplasien und Dermatosen, z.B. Akne oder Psoriasis aufweisen. Nachteil dieser Verbindungen ist ihre geringe therapeutische Breite bezüglich der unter dem Begriff Hypervitaminose A zusammengefaßten Nebenwirkungen.

Es wurde nun gefunden, daß Verbindungen der allgemeinen Formel I

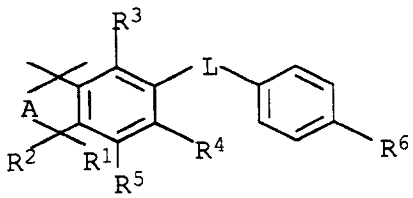

I,

in der:

| | |
|---|---|
| A | einen gegebenenfalls mit einer Methyl, Hydroxyl- oder Oxogruppe substituierten Ethylen- oder Methylenrest, |
| L | einen gesättigten oder ungesättigten 5- oder 6-gliedrigen Heterocyclus mit 1 bis 3 Heteroatomen aus der Gruppe N, O und S, wobei das 2. und 3. Heteroatom ein Stickstoffatom ist, darstellt, der durch eine Hydroxyl-, Mercapto-, $C_{1-6}$-Alkyl oder $C_{1-4}$-Alkanoylgruppe und im Fall des Pyrazol-1,3-diyls sowie des Δ2-Pyrazolin-1,3-diyls in 5-Stellung auch durch $C_{1-4}$-Alkoxycarbonyl substituiert sein kann, |
| $R^1$ und $R^2$ | Wasserstoff oder einen Methylrest, |
| $R^3$ | Wasserstoff, eine Hydroxy- oder eine $C_{1-6}$-Alkoxygruppe, |
| $R^4$ | Wasserstoff, einen $C_{1-4}$-Alkylrest, ein Halogenatom, vorzugsweise Fluor oder eine Methoxygruppe, |
| $R^5$ | Wasserstoff oder eine Methoxygruppe und |
| $R^6$ | Wasserstoff, eine Methyl- oder Nitrilgruppe, eine $C_{2-10}$-Ketalgruppe der Formel |

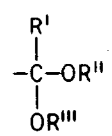

,

wobei R', R'' und R''' Alkylgruppen mit 1 bis 9 C-Atomen darstellen, die Summe der C-Atome von R', R'' und R''' 2 bis 10 beträgt und R' auch Wasserstoff sein kann,

oder die Reste $-CHR^7-OR^8$, $-CHR^7-NR^9R^{10}$, $-COR^{11}$, $-SR^{12}$,

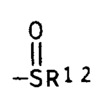 oder 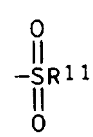

bedeuten, worin

| | |
|---|---|
| $R^7$ | Wasserstoff oder eine $C_{1-4}$-Alkylgruppe |
| $R^8$ | Wasserstoff, eine $C_{1-4}$-Alkyl, $C_{1-20}$-Alkanoyl-, oder eine gegebenenfalls substituierte Benzoylgruppe, |
| $R^9$ und $R^{10}$ | Wasserstoffatome, $C_{1-4}$-Alkyl, $C_{1-6}$-Alkanoyl- oder gegebenenfalls substituierte Benzoylgruppen oder zusammen mit dem Stickstoffatom, an das sie gebunden |

sind, einen heterocyclischen Rest,

$R^{11}$ ein Wasserstoffatom, eine $C_{1-4}$-Alkylgruppe oder die Reste $-OR^{13}$ oder $-NR^{14}R^{15}$ mit $R^{13}$ in der Bedeutung eines Wasserstoffs, einer gegebenenfalls durch Hydroxylgruppen substituierten $C_{1-8}$-Alkylgruppe, einer gegebenenfalls substituierten Aryl- oder gegebenenfalls im Arylteil substituierten Aralkylgruppe, und mit $R^{14}$ und $R^{15}$ in der Bedeutung von Wasserstoffatomen, gegebenenfalls durch eine Hydroxygruppe substituierten $C_{1-6}$-Alkylgruppen, gegebenenfalls substituierten Aralkyl- oder Arylgruppen, oder $R^{14}$ und $R^{15}$ zusammen mit dem Stickstoffatom, an das sie gebunden sind, in der Bedeutung eines heterocyclischen Restes darstellen, und

$R^{12}$ eine $C_{1-4}$-Alkylgruppe bedeutet,

und Verbindungen der allgemeinen Formel Ia

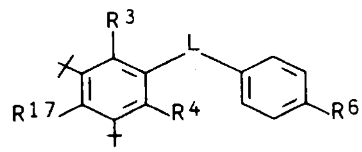

Ia

worin

$R^3$, $R^4$, $R^6$ und L die genannten Bedeutungen haben und

$R^{17}$ = H oder OH ist,

sowie deren physiologisch verträgliche Salze ein verbessertes Wirkprofil, vor allem hinsichtlich der Nebenwirkungen, besitzen.

Als heterocyclische Reste für L kommen vorzugsweise die folgenden Strukturen in Betracht:

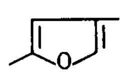 , 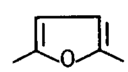 , 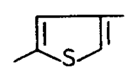 , 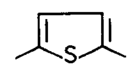

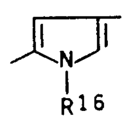 , 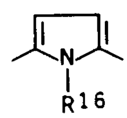 , 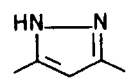 , 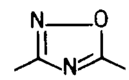

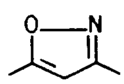 , 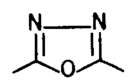 , 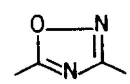 , 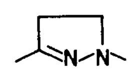

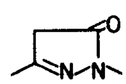 = 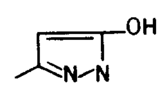 , 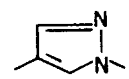 , 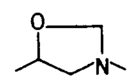

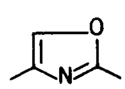 , 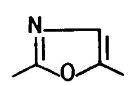 , 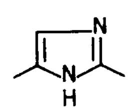 , 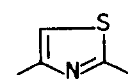

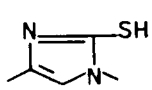 , 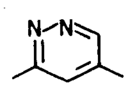 , 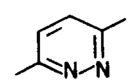 , 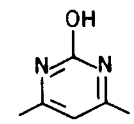

wobei

$R^{16}$ für Wasserstoff, einen $C_{1-6}$-Alkylrest oder einen $C_{1-4}$-Alkanoylrest steht.

Als heterocyclische Reste $-NR^9R^{10}$ und $-NR^{14}R^{15}$ kommen insbesondere Pyrrolidino-, Piperidino- und Morpholino-Reste in Betracht. Bevorzugte Substituenten der Benzoylgruppe ($R^8$,$R^9$,$R^{10}$) sind die Methoxy-, Nitro- und Methylgruppen sowie Halogenatome, insbesondere Chlor oder Brom. Bevorzugt als Arylrest

($R^{13}$,$R^{14}$,$R^{15}$) ist die Phenylgruppe, die gegebenenfalls durch eine Methyl-, Methoxy- oder Nitrogruppe substituiert ist. Als Aralkylgruppe ($R^{13}$,$R^{14}$,$R^{15}$) ist die Benzylgruppe bevorzugt, die im Arylteil insbesondere durch eine Methyl- oder Methoxygruppe oder ein Halogenatom, vorzugsweise Chlor oder Brom, substituiert sein kann.

Die erfindungsgemäßen Verbindungen der Formel I lassen sich nach im Prinzip bekannten Methoden der Heterocyclensynthese herstellen. Eine Übersicht findet sich z.B. in "Comprehensive Heterocyclic Chemistry" (Eds. A.R. Katritzky and C.W. Rees), Vol. 1 - 8, Pergamon Press, 1984.

Vorzugsweise wurden folgende Verfahren benutzt:

a) Umsetzung einer 1,3-Diketoverbindung der Formel II

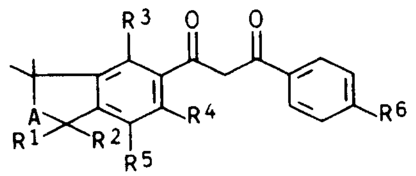

II,

worin

A und $R^1$ - $R^6$ die oben angegebenen Bedeutungen haben, mit Hydrazin, Hydroxylamin oder Harnstoff zu dem entsprechenden Pyrazol, Isoxazol oder 2-Hydroxypyrimidin; diese Umsetzung läßt sich offensichtlich variieren, beispielsweise indem der durch $R^6$ substituierte Phenylkern von vornherein mit einem N-Atom des Hydrazins, Hydroxylamins oder des Harnstoffs verknüpft ist. In Formel II sitzt dann an seiner Stelle z.B. ein Niederalkoxy-Rest.

b) Umsetzung einer 1,4-Diketoverbindung der Formel III oder eines Ketals der Formel IV

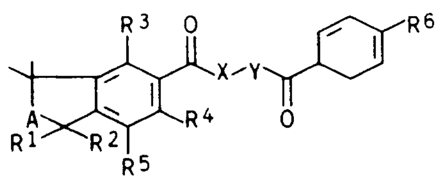

III,

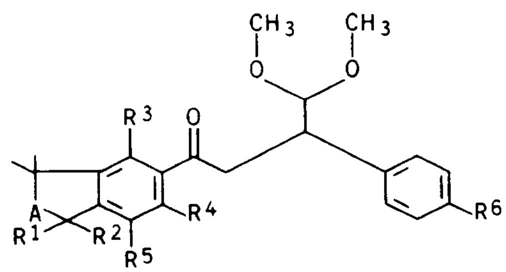

IV,

worin

A und $R^1$ - $R^6$ die oben angegebenen Bedeutungen haben, und

X und Y in Formel III jeweils für $-CH_2-$ oder -NH- stehen, mit dehydratisierenden Mitteln, gegebenenfalls zusammen mit Sulfiden bzw. Schwefelwasserstoff, Ammoniak bzw. Ammoniumsalzen, prim. Aminen, Hydrazin zu dem entsprechenden Furan, Thiophen, Pyrrol, Pyridazin, Oxazol oder 1,3,4-Oxadiazol;

c) Umsetzung eines Carbonsäurederivates der Formel V bzw. VI

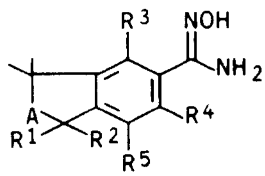

V,

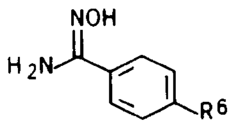

VI,

worin

A und $R^1$ bis $R^6$ die oben angegebenen Bedeutungen haben,

mit einem anderen Carbonsäurederivat der Formel VII bzw. VIII,

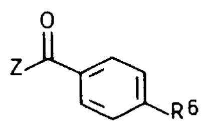

VII,

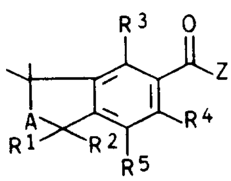

VIII,

worin

A und $R^1$ bis $R^6$ die oben angegebenen Bedeutungen haben, und

Z für Chlor, eine $C_{1-4}$-Alkoxy- oder Hydroxygruppe steht,

unter cyclisierenden, in üblicher Weise dehydratisierenden Reaktionsbedingungen umsetzt;

d) 1,3-Dipolare Cycloaddition eines Nitriloxids der Formel IX

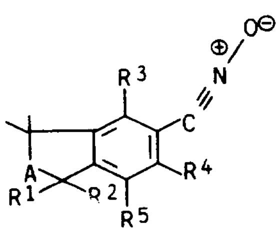

IX,

worin

A und $R^1$-$R^5$ die oben angegebenen Bedeutungen haben, an ein Monoarylacetylen der Formel X

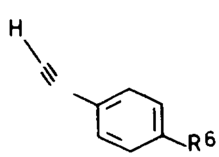

X,

worin

$R^6$ die oben angegebenen Bedeutungen hat, zu Isoxazolen der Formel I.

e) Methoden, die die nach den unter a) - d) beschriebenen oder nach anderen Verfahren hergestellten Verbindungen durch an sich bekannte Abwandlung des Restes $R^6$ in weitere erfindungsgemäße Verbindungen der Formel I überführen.

Die Verfahren gemäß a) - c) sind im Prinzip bekannte Kondensationsreaktionen, die teils ohne den Zusatz eines Katalysators ablaufen, vorzugsweise jedoch mittels einer Base oder Säure katalysiert werden, die vorteilhafterweise zugleich auch als Dehydratisierungsmittel eingesetzt wird. Als solche Mittel finden vorzugsweise Verwendung: Mineralsäuren wie Salzsäure, Schwefelsäure oder Polyphosphorsäure, organische Säuren wie Eisessig, Lewissäuren wie Phosphoroxytrichlorid, Phosphorpentachlorid, Tetraphosphordekaoxid, Thionylchlorid, Bortrifluoriddiethyletherat, oder auch Basen wie Natriumhydroxid, Kaliumhydroxid, Kaliumcarbonat, Natriumhydrogencarbonat, Natriummethanolat oder Kalium-tert.-butanolat.

Die Umsetzungen werden vorteilhafterweise in einem geeigneten Lösungsmittel bei Temperaturen von 0 - 200 °C durchgeführt, vorzugsweise bei der Rückflußtemperatur des verwendeten Lösungsmittels. Als Lösungsmittel kommen in Frage vor allem Alkohole wie Methanol, Ethanol, Isopropanol oder n-Butanol, aromatische Kohlenwasserstoffe wie Benzol, Toluol oder Xylol, aprotische dipolare Lösungsmittel wie Dimethylformamid oder Dimethylsulfoxid oder chlorierte Kohlenwasserstoffe wie Methylenchlorid, Chloroform, 1,2-Dichlorethan oder 1,2,3-Trichlorethan.

Die in dem Verfahren gemäß d) verwendeten Nitriloxide werden vorteilhafterweise in situ aus den entsprechenden Oximen der Formel XI durch Oxidation hergestellt.

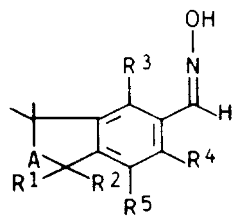

XI,

Als Oxidationsmittel verwendet man Chlor, Nitrosylchlorid, N-Chlor- oder N-Bromsuccinimid, Bleitetraacetat oder wäßrige Hypohalogenitlösungen, vorzugsweise Natriumhypochloritlösung. Gegebenenfalls wird eine Base wie Natronlauge oder Triethylamin zugesetzt. Man führt die Umsetzung zweckmäßigerweise in einem geeigneten Lösungsmittel, z. B. Methylenchlorid oder Dimethylformamid, bei einer Temperatur zwischen -20 und +70 °C, vorzugsweise bei 20 - 35°C, durch.

Zu den Methoden gemäß e) gehören beispielsweise:

Benzoesäureester oder Benzonitrile der allgemeinen Formel I, in der $R^6$ eine Carboalkoxy- bzw. eine Nitrilgruppe bedeutet, lassen sich in die freien Carbonsäuren und ihre physiologisch verträglichen Salze durch Verseifung überführen. Die Verseifung wird vorzugsweise in einem Gemisch eines niederen aliphatischen Alkohols wie Methanol, Ethanol, Propanol, Isopropanol oder n-Butanol mit Wasser in Gegenwart eines im Überschuß eingesetzten Alkalihydroxids, vorzugsweise Natrium- oder Kaliumhydroxid, durchgeführt.

Die erfindungsgemäßen Amide können in an sich bekannter Weise hergestellt werden, indem man die entsprechenden Benzoesäuren zunächst in carbonylaktivere Derivate überführt, z. B. in die Säurehalogenide, -azide, -imidazolide oder -anhydride, und diese mit Aminen $HNR^{14}R^{15}$ behandelt.

Eine Carbonsäure, ein Carbonsäureester, ein Carbonsäureamid oder ein Nitril der Formel I kann in an sich bekannter Weise zu den entsprechenden Alkoholen bzw. Aminen reduziert werden. Vorteilhaft wird die Reaktion mit Hilfe eines Metallhydrids oder Alkalimetallhydrids in Gegenwart eines geeigneten Lösungsmittels durchgeführt. Als Metallhydride werden vorzugsweise komplexe Metallhydride wie Lithiumaluminium- oder Lithiumborhydrid oder Diisobutylaluminiumhydrid eingesetzt. Bevorzugte Lösungsmittel sind Ether wie Diethylether, Tetrahydrofuran, Dioxan oder 1,2-Dimethoxyethan.

Ein Alkohol oder Amin der Formel I kann in an sich bekannter Weise mit einem Alkanoyl- oder Aroylchlorid oder -anhydrid zu dem entsprechenden Ester bzw. Amid acyliert oder mit einem Alkylhalogenid, vorzugsweise einem Alkylbromid oder -iodid, zu dem entsprechenden Ether oder höheralkylierten Amin alkyliert oder mit geeigneten Oxidationsmitteln, wie Mangan(IV)-oxid, zu dem entsprechenden Aldehyd oxidiert werden.

Ein Aldehyd der Formel I kann auch durch Reduktion des entsprechenden Nitrils der Formel I mit Diisobutylaluminiumhydrid in einem Lösungsmittel, vorzugsweise Toluol, Hexan oder Tetrahydrofuran, in einem Temperaturbereich zwischen -40 °C und Raumtemperatur erhalten werden.

Die erfindungsgemäßen Verbindungen und ihre physiologisch verträglichen Salze können aufgrund ihrer pharmakologischen Eigenschaften bei der topischen und systemischen Therapie und auch Prophylaxe von Praekanzerosen und Karzinomen der Haut, der Schleimhäute und inneren Organe sowie bei der topischen und systemischen Therapie von Akne, Psoriasis und anderer mit pathologisch veränderter Verhornung einhergehenden dermatologischen Erkrankungen, insbesondere Ichthyosis, Dariersche Krankheit, Flechte, Leukoplakie, aber auch gegen Ekzeme, Vitiligo, ferner gegen trockene Augen und andere Corneopathien sowie zur Behandlung von rheumatischen Erkrankungen, insbesondere solchen entzündlicher oder degenerativer Art, die Gelenke, Muskeln, Sehnen und andere Teile des Bewegungsapparates befallen, verwendet werden. Ein bevorzugtes Indikationsgebiet ist neben der Therapie von dermatologischen Erkrankungen und durch Sonnenlichteinwirkung oder iatrogen, z.B. durch Corticosteroide induzierte Atrophie, bedingten Hautschädigungen, die Prophylaxe gegen Praekanzerosen und Tumoren.

Als Kosmetikum können die erfindungsgemäßen Verbindungen eingesetzt werden zur Therapie von Lichtschäden der Haut (Versprödung, nicht altersbedingte, sondern durch übermäßige UV- oder Sonnenlicht-Einwirkung entstehende Runzeln und Fältchen), ferner gegen Warzen.

Die pharmakologischen Wirkungen können beispielsweise in den nachfolgenden Testmodellen aufgezeigt werden. Die erfindungsgemäßen Verbindungen heben an Hamstertrachealgewebe in vitro die nach Vitamin-A-Mangel einsetzende Keratinisierung auf. Die Keratinisierung gehört zur Frühphase der Carcinogenese, die in einer ähnlichen Technik in vivo nach der Initation durch chemische Verbindungen, durch energetische Strahlung oder nach viraler Zelltransformation durch die erfindungsgemäßen Verbindungen der Formel I inhibiert wird. Diese Methodik kann aus Cancer Res. 36, 964 - 972 (1972) oder aus Nature 250, 64 - 66 (1974) und Nature 253, 47 - 50 (1975) entnommen werden.

Darüberhinaus wird durch die erfindungsgemäßen Verbindungen die Proliferation bestimmter maligne veränderter Zellen inhibiert. Diese Methodik kann aus J. Natl. Cancer Inst. 60, 1035 - 1041 (1978), Experimental Cell Research 117, 15 - 22 (1978) und Proc. Natl. Acad. Sci. USA 77, 2937 -2940 (1980) entnommen werden.

Die antiarthritische Wirkung der erfindungsgemäßen Verbindungen kann in üblicher Weise im Tierexperiment im Adjuvans-Arthritis- oder Streptokokkenzellwandinduzierten-Arthritis-Modell bestimmt werden. Die dermatologische Aktivität, beispielsweise für die Behandlung von Akne, kann u. a. durch die komedolytische Aktivität und die Fähigkeit nachgewiesen werden, die Anzahl der Zysten im Modell der Rhino-Maus zu reduzieren.

Diese Methode ist von L. H. Kligman et al. in The Journal of Investigative Dermatology 73, 354 - 358 (1978) beschrieben. Als weiteres Maß für die dermatologische Aktivität kann die Reduktion der Talgdrüsen und die damit einhergende verminderte Talgproduktion am Seitenorgan des Hamsters dienen. Diese Methodik ist von E. C. Gomez in J. Am. Dermatol. 6, 746 - 750 (1982) beschrieben.

Ferner kann die durch die erfindungsgemäßen Verbindungen erzielbare Reversion von Hautschäden, welche durch UV-Licht ausgelöst werden, in Tiermodellen bestimmt werden. Diese Methode ist von L. H. Kligman et al. beschrieben in Connect. Tissue Res. 12, 139 - 150 (1984) und in J. Am. Acad. Dermatol. 15, 779 - 785 (1986).

Dementsprechend sind ein weiterer Gegenstand der Erfindung therapeutische Mittel zur topischen und systemischen Anwendung sowie kosmetische Mittel, die eine Verbindung der Formel I neben üblichen Trägerstoffen oder Verdünnungsmitteln und den in üblicher Weise verwendeten pharmazeutischtechnischen bzw. kosmetischen Hilfsstoffen, entsprechend der gewünschten Applikationsart und mit einer zur Anwendung geeigneten Dosierung, enthalten.

Die Mittel können peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen und kosmetischen Mittel können die erfindungsgemäß zu verwendenden Verbindungen bei lokaler Anwendung in 0,0001 bis 1 %iger Konzentration, bevorzugt in 0,001 bis 0,1 %iger Konzentration, und bei systemischer Anwendung vorzugsweise in einer Einzeldosis von 0,1 bis 50 mg enthalten und täglich in einer oder mehreren Dosierungen je nach Art und Schwere der Erkrankungen verabreicht werden.

Die Arzneimittel und Kosmetika der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt.

Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie

Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch überziehen von analog den Tabletten hergestellten Kernen mit üblicherweise in Drageeüberzügen verwendeten Mitteln, beispielsweise Polyvinylpyrrolidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z.B. Aromastoffe wie Vanillin oder Orangenextrakt, enthalten. Sie können außerdem Suspendierhilfsstoffe wie Natriumcarboxymethylcellulose oder Konservierungsstoffe wie p-Hydroxybenzoate, enthalten. Wirkstoffe enthaltende Kapseln können beispielsweise hergestellt werden, indem man den Wirkstoff mit einem inerten Träger, wie Milchzucker oder Sorbit, mischt und in Gelatinekapseln einkapselt.

Zweckmäßige übliche Bestandteile von kosmetischen und pharmazeutischen Zubereitungen für die topische Anwendung sind beispielsweise:
anionische, kationische sowie nichtionische Emulgatoren und Emulsionsstabilisatoren, die gleichzeitig Konsistenzgeber oder Gelbildner sein können, wie Polyvinylpyrrolidon, Fettalkohole, Glycerinmonostearat, Polyacrylsäuren, Cellulosederivate und Ethylenoxid-Propylenoxid-Blockpolymere,
feste oder flüssige Ölkomponenten bzw. Fettstoffe mineralischer, pflanzlicher oder tierischer Herkunft, synthetische Esteröle, wie Glyceroltriester und Isopropylmyristat,
hydrophile Komponenten, wie Glycerin, Polyethylenglykol und Propylenglykol.

Weiterhin können als Inhaltsstoffe von Kosmetika genannt werden beispielsweise Lichtschutzmittel, Bräunungsmittel, Konservierungsmittel, Antioxidantien, Pigmente, Farbstoffe, ätherische Öle und Parfümöle, Vitamine, Pflanzenextrakte, Collagen usw. Diese Stoffe können beispielsweise dem CTFA, Cosmetic Ingredient Dictionary, 3. Auflage, Washington 1982, entnommen werden.

Einige der erfindungsgemäßen Verbindungen weisen ein acides Wasserstoffatom auf und können daher mit Basen in üblicher Weise in ein physiologisch verträgliches, gut wasserlösliches Salz übergeführt werden. Geeignete Salze sind beispielsweise Ammonium-, Alkalimetallsalze, insbesondere des Natriums, Kaliums und Lithiums, und Erdalkalimetallsalze, insbesondere des Calciums oder Magnesiums, sowie Salze mit geeigneten organischen Basen, wie $C_{1-6}$-Alkylaminen, z. B. Methylamin, Ethylamin oder Cyclohexylamin, oder mit substituierten $C_{1-6}$-Alkylaminen, insbesondere hydroxysubstituierten Alkylaminen, wie Diethanolamin, Triethanolamin oder Tris(hydroxymethyl)aminomethan, sowie mit Piperidin oder Morpholin.

Gegebenenfalls werden die erhaltenen erfindungsgemäßen Amine der Formel I nach bekannter Verfahrensweise in das Säureadditionssalz einer physiologisch verträglichen Säure übergeführt. Als übliche physiologisch verträgliche organische oder anorganische Säure kommen beispielsweise in Betracht Salzsäure, Bromwasserstoffsäure, Phosphorsäure oder Schwefelsäure und als organische Säuren beispielsweise Maleinsäure, Fumarsäure, Milchsäure, Weinsäure, Äpfelsäure, Zitronensäure, Salicylsäure, Adipinsäure oder Benzoesäure. Weitere können aus "Fortschritte der Arzneimittelforschung" Band 10, Seiten 224 - 225, Birkhäuser Verlag, Basel und Stuttgart, 1966, entnommen werden.

Beispiel 1

4-(4-Carbomethoxyphenyl)-2-(5,6,7,8-tetrahydro-5,5,8,8-tetramethyl-2-naphthalenyl)furan

3-(4-Carbomethoxyphenyl)-1-(5,6,7,8-tetrahydro-5,5,8,8-tetramethyl-2-naphthalenyl)-2-propen-1-on:

138 g (0,6 mol) 6-Acetyl-1,2,3,4-tetrahydro-1,1,4,4-tetramethylnaphthalin und 98,5 g (0,6 mol) 4-Formylbenzoesäuremethylester wurden in einem Gemisch aus 16 g Natriumhydroxid und 690 ml Methanol 16 h bei Raumtemperatur gerührt. Das ausgefallene Kristallisat wurde abgesaugt, und nach Trocknen verblieben 132 g 3-(4-Carbomethoxyphenyl)-1-(5,6,7,8-tetrahydro-5,5,8,8-tetramethyl-2-naphthalenyl)-2-propen-1-on, Schmp. 89 - 91 °C.

3-(4-Carbomethoxyphenyl)-4-dimethoxyl-1-(5,6,7,8-tetrahydro-5,5,8,8-tetramethyl-2-naphthalenyl)-1-butanon:

47,3 g (126 mmol) 3-(4-Carbomethoxyphenyl)-1-(5,6,7,8-tetrahydro-5,5,8,8-tetramethyl-2-naphthalenyl)-2-propen-1-on, 360 ml Nitromethan und 4 g 40%ige methanolische Benzyltrimethylammoniumhydroxidlösung (Triton B) wurden 5 h bei Raumtemperatur gerührt. Danach versetzte man mit 850 ml Ether, wusch nacheinander mit 2 N Salzsäure, 2x mit ges. Natriumhydrogencarbonatlösung, 2x mit Wasser und 1x mit ges. Natriumchloridlösung, trocknete über Natriumsulfat und engte ein. Es verblieben 51,7 g eines öligen Rückstands; 45 g davon wurden in 122 ml Methylenchlorid und 122 ml Tetrahydrofuran gelöst und bei -35°C zu 245 ml einer 1 M Natriummethanollösung in Methanol zugetropft.

Diese Lösung wurde bei -35 °C zu einem separat hergestellten Gemisch aus 245 ml konz. Schwefelsäure und 920 ml Methanol (man tropft die Schwefelsäure bei -35 °C in das Methanol ein) zugetropft. Man rührte bei -35 °C 0,5 h nach, ließ dann auf Raumtemperatur kommen und nochmals 1 h nachrühren. Zur Aufarbeitung wurden 1,5 l Methylenchlorid zugegeben, unter externer Kühlung und Rühren Wasser zugegeben und die Phasen getrennt. Die organische Phase wurde mit 2 N Natronlauge, dann mit ges. Natriumchloridlösung gewaschen, über Natriumsulfat getrocknet und eingeengt. Man erhielt so 40 g 3-(4-Carbomethoxyphenyl)-4-dimethoxy-1-(5,6,7,8-tetrahydro-5,5,8,8-tetramethyl-2-naphthalenyl)-1-butanon als öliges Rohprodukt, das ohne weitere Reinigung weiter umgesetzt wurde. Die Struktur wurde durch H-NMR-Spektroskopie abgesichert.

4-(4-Carbomethoxyphenyl)-2-(5,6,7,8-tetrahydro-5,5,8,8-tetramethyl-2-naphthalenyl)furan:

10 g (22 mmol) 3-(4-Carbomethoxyphenyl)-4-dimethoxy-1-(5,6,7,8-tetrahydro-5,5,8,8-tetramethyl-2-naphthalenyl)-1-butanon wurden in 50 g konz. Schwefelsäure 12 h bei 25°C gerührt. Danach gab man das Reaktionsgemisch auf Eis/Wasser und saugte den ausgefallenen Niederschlag ab. Nach Trocknen und Umkristallisieren aus n-Heptan erhielt man 3,8 g der Titelverbindung, Schmp. 109 - 111°C.

Beispiel 2

4-(4-Carbomethoxyphenyl)-2-(5,6,7,8-tetrahydro-5,5,8,8-tetramethyl-2-naphthalenyl)thiophen:

5,5 g (12 mmol) 3-(4-Carbomethoxyphenyl)-4-dimethoxy-1-(5,6,7,8-tetrahydro-5,5,8,8-tetramethyl-2-naphthalenyl)-1-butanon (Herstellung s. Beispiel 1) und 2,7 g Tetraphosphordekasulfid wurden in 100 ml Xylol 2 h unter Rückfluß erhitzt. Dann wurde das Lösungsmittel abdestilliert, und der Rückstand wurde säulenchromatographisch (Kieselgel; Methylenchlorid/n-Heptan 1 : 1) gereinigt. Man erhielt so 1,4 g der Titelverbindung, Schmp. 108 - 110°C.

Beispiel 3

4-(4-Carbomethoxyphenyl)-2-(5,6,7,8-tetrahydro-5,5,8,8-tetramethyl-2-naphthalenyl)pyrrol

4,0 g (9 mmol) 3-(4-Carbomethoxyphenyl)-4-dimethoxy-1-(5,6,7,8-tetrahydro5,5,8,8-tetramethyl-2-naphthalenyl)-1-butanon (Herstellung s. Beispiel 1) und 3,4 g (44 mmol) Ammoniumacetat wurden in 100 ml Essigsäure 2 h unter Rückfluß erhitzt. Nach dem Abkühlen gab man auf Wasser und saugte den entstandenen Niederschlag ab. Nach Trocknen und Umkristallisieren aus n-Heptan erhielt man 2,3 g der Titelverbindung, Schmp. 174 - 175°C.

Beispiel 4

2-(5,6,7,8-Tetrahydro-5,5,8,8-tetramethyl-2-naphthalenyl)-4-(4-tolyl)furan

1-(5,6,7,8-tetrahydro-5,5,8,8-tetramethyl-2-naphthalenyl)-3-(4-tolyl)-2-propen-1-on:

Zu einer Lösung von 50 g (0,22 mol) 6-Acetyl-1,2,3,4-tetrahydro-1,1,4,4-tetramethylnaphthalin und 26,1 g (0,22 mol) 4-Tolylaldehyd in 250 ml Methanol wurden 10 g 50 %ige Natronlauge zugetropft. Man ließ über Nacht rühren, gab danach auf Eis/Wasser und ließ mehrere Stunden stehen. Das ausgefallene Kristallisat wurde abgesaugt, und nach Trocknen erhielt man 70 g 1-(5,6,7,8-Tetrahydro-5,5,8,8-tetramethyl-2-naphthalenyl)-3-(4-tolyl)-2-propen-1-on, Schmp. 153 - 154°C.

4-Dimethoxy-1-(5,6,7,8-tetrahydro-5,5,8,8-tetramethyl-2-naphthalenyl)-3-(4-tolyl)-1-butanon:

Analog Beispiel 1 erhielt man aus 70 g (90 mmol) 1-(5,6,7,8-Tetrahydro-5,5,8,8-tetramethyl-2-naphthalenyl)-3-(4-tolyl)-2-propen-1-on 68 g 4-Dimethoxy-1-(5,6,7,8-tetrahydro-5,5,8,8-tetramethyl-2-aphthalenyl)-3-(4-tolyl)-1-butanon als Öl, das ohne Reinigung weiter umgesetzt wurde. Die Struktur wurde durch H-NMR-Spektroskopie abgesichert.

2-(5,6,7,8-Tetrahydro-5,5,8,8-tetramethyl-2-naphthalenyl)-4-(4-tolyl)furan:

Analog Beispiel 1 erhielt man aus 4,2 g (11 mmol) 4-Dimethoxy-1-(5,6,7,8-tetrahydro-5,5,8,8-tetramethyl-2-naphthalenyl)-3-(4-tolyl)-1-butanon nach säulenchromatographischer Reinigung (Kieselgel; Methylenchlorid) 2,2 g der Titelverbindung, Schmp. 155 -157°C (aus Isopropanol).

Beispiel 5

2-(5,6,7,8-Tetrahydro-5,5,8,8-tetramethyl-2-naphthalenyl)-4-(4-tolyl)thiophen

Analog Beispiel 2 erhielt man aus 10 g (25 mmol) 4-Dimethoxy-1-(5,6,7,8-tetrahydro-5,5,8,8-tetramethyl-2-naphthalenyl)-3-(4-tolyl)-1-butanon (Herstellung s. Beispiel 4) nach säulenchromatographischer Reinigung (Kieselgel; Methylenchlorid) und Umkristallisation aus n-Heptan 3,5 g der Titelverbindung, Schmp. 135 -136 °C.

Beispiel 6

2-(5,6,7,8-Tetrahydro-5,5,8,8-tetramethyl-2-naphthalenyl)-4-(4-tolyl)pyrrol

Analog Beispiel 3 erhielt man aus 5,0 g (12 mmol) 4-Dimethoxy-1-(5,6,7,8-tetrahydro-5,5,8,8-tetramethyl-2-naphthalenyl)-3-(4-tolyl)-1-butanon (Herstellung s. Beispiel 4) 2,1 g der Titelverbindung, Schmp. 190 - 192 ° C.

Beispiel 7

2-(4-Carbomethoxyphenyl)-5-(5-6,7,8-tetrahydro-5,5,8,8-tetramethyl-2-naphthalenyl)furan

Zu 310 ml (0,48 mol) einer 1,55 molaren Lösung von Vinylmagnesiumchlorid in Tetrahydrofuran tropfte man bei 0 °C eine Lösung von 86,4 g (0,4 mol) 5,6,7,8-Tetrahydro-5,5,8,8-tetramethyl-2-naphthaldehyd in 480 ml Tetrahydrofuran. Man ließ 10 min. nachrühren und tropfte dann, wiederum bei 0°C, 60 g (0,4 mol) Acetanhydrid zu. Man ließ 15 min. nachrühren, und versetzte dann vorsichtig mit ges. Natriumchloridlösung. Man extrahierte 2x mit Ether, wusch die organischen Extrakte mit Wasser und ges. Natriumcarbonatlösung, trocknete über Natriumsulfat und engte ein. Es verblieben 100,7 g 1-0-Acetyl-1-(5,6,7,8-Tetrahydro-5,5,8,8-tetramethyl-2-naphthalenyl)-2-propen-1-ol als Öl.

Zu einer Lösung von 120 g (0,42 mol) davon und 75 g (0,42 mol) 4-(Hydroximino)-methylbenzoesäuremethylester in 500 ml Methylenchlorid wurden bei 0°C 300 g 10 %ige wäßrige Natriumhypochloritlösung zugetropft. Man ließ über Nacht nachrühren. Danach versetzte man mit weiteren 500 ml Methylenchlorid, trennte die Phasen und extrahierte die wäßrige Phase nochmals mit Methylenchlorid. Die vereinigten organischen Phasen wurden mit Wasser gewaschen, über Natriumsulfat getrocknet und eingeengt. Der Rückstand (179,5 g) wurde in 1,7 l Tetrahydrofuran aufgenommen. Zu dieser Lösung gab man 40 ml 30 %ige methanolische Natriummethanolatlösung und ließ über Nacht rühren. Das Tetrahydrofuran wurde weitgehend am Rotationsverdampfer entfernt und das verbleibende Öl in Ether aufgenommen. Man wusch die Etherlösung 2x mit Wasser, trocknete über Natriumsulfat und engte ein.

Der Rückstand (118 g) wurde in einem Gemisch aus 1000 ml Tetrahydrofuran, 170 ml Eisessig und 90 ml Wasser mit 40 g Raney-Nickel bei 50 bar Wasserstoffdruck im Autoklaven über 48 h bei Raumtemp. hydriert. Das Reaktionsgemisch wurde danach über Celite zur Entfernung des Katalysators filtriert und das Filtrat eingeengt. Der Rückstand (150 g) wurde in einer Mischung von 750 ml Methylenchlorid und 750 ml 10 %iger Salzsäure 3 h bei Raumtemp. gerührt. Danach wurden die Phasen getrennt, die organische Phase wurde über Natriumsulfat getrocknet und eingeengt. Der Rückstand wurde mit n-Heptan heiß extrahiert und der n-Heptanextrakt eingeengt. Das verbleibende Öl wurde aus Methanol kristallisiert und man erhielt so 24,2 g der Titelverbindung, Schmp. 141 - 142°C.

Beispiel 8

2-[(4-Hydroxymethyl)phenyl]-5-(5,6,7,8-tetrahydro-5,5,8,8-tetramethyl-2-naphthalenyl)furan

3 g (7,7 mmol) 2-(4-Carbomethoxyphenyl]-5-(5,6,7,8-tetrahydro-5,5,8,8-tetramethyl-2-naphthalenyl)furan (Beispiel 7), 1,22 g Lithiumchlorid und 1,08 g Natriumborhydrid wurden in einem Gemisch aus 10 ml Tetrahydrofuran und 20 ml Ethanol über Nacht bei Raumtemperatur gerührt. Danach gab man Eis zu, stellte mit 10 %iger Zitronensäure auf pH 4 ein und entfernte das Tetrahydrofuran am Rotationsverdampfer. Der Rückstand wurde mit Methylenchlorid extrahiert. Die organische Phase wurde mit Natriumsulfat getrocknet und eingeengt. Nach Umkristallisation aus n-Heptan erhielt man 1,4 g der Titelverbindung, Schmp. 124 - 125°C.

Beispiel 9

2-(4-Carbomethoxyphenyl)-5-(5,6,7,8-tetrahydro-5,5,8,8-tetramethyl-2-naphthalenyl)thiophen

6-(3-Chlorpropionyl)-1,2,3,4-tetrahydro-1,1,4,4-tetramethylnaphthalin:

Zu einer Lösung von 70 g (0,55 mol) 3-Chlorpropionsäurechlorid in 200 ml Methylenchlorid wurden bei 0 - 5 °C 73,5 g (0,55 mol) wasserfreies Aluminiumchlorid portionsweise zugegeben und danach bei derselben Temperatur eine Lösung von 94 g (0,5 mol) 1,2,3,4-Tetrahydro-1,1,4,4-tetramethylnaphthalin in 200 ml Methylenchlorid zugetropft. Man ließ über Nacht bei Raumtemperatur rühren, goß auf 1 l Eis/Wasser und extrahierte dreimal mit je 300 ml Methylenchlorid. Die vereinigten organischen Phasen wurden mit ges. Natriumhydrogencarbonatlösung und Wasser gewaschen, über Magnesiumsulfat getrocknet und eingeengt. Es verblieben 138 g 6-(3-Chlorpropionyl)-1,2,3,4-tetrahydro-1,1,4,4-tetramethylnaphthalin als Öl. Die Struktur wurde durch H-NMR-Spektroskopie abgesichert.

1-(4-Carbomethoxyphenyl)-4-(5,6,7,8-tetrahydro-5,5,8,8-tetramethyl-2-naphthalenyl)-1,4-butandion

5,6 g (0,02 mol) 6-(3-Chlorpropionyl)-1,2,3,4-tetrahydro-1,1,4,4-tetramethylnaphthalin und 3,3 ml (0,024 mol) Triethylamin wurden in 30 ml Dimethylformamid 1 h bei Raumtemperatur gerührt. Danach wurde eine Lösung von 3,6 g (0,025 mol) 4-Formylbenzoesäuremethylester und 1 g 3-Benzyl-5-(2-hydroxyethyl)-4-methylthiazoliumchlorid in 10 ml Dimethylformamid zugetropft. Man ließ 1 h nachrühren und goß danach auf Eis/Wasser, extrahierte 2x mit Essigester, wusch die vereinigten organischen Extrakte mehrmals mit Wasser, trocknete über Magnesiumsulfat und engte ein. Aus dem Rückstand (6,4 g) erhielt man nach Umkristallisation aus Ethanol 3,7 g 1-(4-Carbomethoxyphenyl)-4-(5,6,7,8-tetrahydro-5,5,8,8-tetramethyl-2-naphthalenyl)-1,4-butandion, Schmp. 139 - 141°C.

2-(4-Carbomethoxyphenyl)-5-(5,6,7,8-tetrahydro-5,5,8,8-tetramethyl-2-naphthalenyl)thiophen:

12,1 g (30 mmol) 1-(4-Carbomethoxyphenyl)-4-(5,6,7,8-tetrahydro-5,5,8,8-tetramethyl-2-naphthalenyl)-1,4-butandion und 13,4 g (00 mmol) Tetraphosphordekasulfid wurden in 150 ml o-Xylol 10 min bei 80 °C unter Stickstoff erhitzt. Nach dem Abkühlen verdünnte man mit Essigester, filtrierte, wusch das Filtrat mit 4 x 100 ml Wasser, trocknete über Natriumsulfat und engte ein. Der Rückstand wurde säulenchromatographisch (Kieselgel; n-Heptan + steigende Anteile Essigester) aufgereinigt. Aus diesem Rohprodukt erhielt man nach Umkristallisation aus Ethanol 8,5 g der Titelverbindung, Schmp. 126 -129°C.

Beispiel 10

2-(4-Carbomethoxyphenyl)-5-(5,6,7,8-tetrahydro-5,5,8,8-tetramethyl-2-naphthalenyl)pyrrol

8,0 g (20 mmol) 1-(4-Carbomethoxyphenyl)-4-(5,6,7,8-tetrahydro-5,5,8,8-tetramethyl-2-naphthalenyl)-1,4-butandion (Herstellung s. Beispiel 9) und 5,5 g (0,1 mol) Ammoniumchlorid wurden in 150 ml trockenem Dimethylformamid 6 h bei 150 °C erhitzt. Nach dem Abkühlen gab man auf Wasser, saugte die ausgefallenen Kristalle ab, wusch mehrmals mit Wasser nach und trocknete im Stickstoffstrom. Nach säulenchromatographischer Reinigung (Kieselgel; n-Heptan + steigende Anteile Essigester) und Umkristallisation aus Ethanol erhielt man 3,6 g der Titelverbindung, Schmp. 176 - 179°C.

Beispiel 11

2-(4-Carbomethoxyphenyl)-1-methyl-5-(5,6,7,8-tetrahydro-5,5,8,8-tetramethyl-2-naphthalenyl)pyrrol

Analog Beispiel 10 erhielt man aus 8,0 g (20 mmol) 1-(4-Carbomethoxyphenyl)-4-(5,6,7,8-tetrahydro-5,5,8,8-tetramethyl-2-naphthalenyl)-1,4-butandion (Herstellung s. Beispiel 9) und 6,7 g (0,1 mol) Methylammoniumchlorid nach Umkristallisation aus Methanol 5,5 g der Titelverbindung, Schmp. 104 - 105°C.

Beispiel 12

3-(4-Carbomethoxyphenyl)-5-(5,6,7,8-tetrahydro-5,5,8,8-tetramethyl-2-naphthalenyl)pyrazol

1-(4-Carbomethoxyphenyl)-3-(5,6,7,8-tetrahydro-5,5,8,8-tetramethyl-2-naphthalenyl)-1,3-propandion:

Zu einer Suspension von 3,6 g (0,15 mol) Natriumhydrid in 21 ml Toluol wurde unter Stickstoff bei 100 °C eine Lösung von 19,4 g (0,1 mol) Terephthalsäuredimethylester und 23 g (0,1 mol) 6-Acetyl-1,2,3,4-tetrahydro-1,1,4,4-tetramethylnaphthalin in einem Gemisch aus 80 ml Toluol und 20 ml Dimethoxyethan zugetropft. Man ließ 5 h unter Rückfluß rühren. Nach dem Abkühlen wurde das Reaktionsgemisch mit 50 ml Wasser versetzt, mit halbkonzentrierter Salzsäure angesäuert, auf 500 ml Wasser gegossen und mit Chloroform extrahiert. Bereits ausgefallenes Produkt wurde abgesaugt, die Chloroformphase über Natriumsulfat getrocknet und eingeengt. Der Rückstand und das vorher abgesaugte Rohprodukt wurden zusammengegeben und mehrmals mit heißem Methanol extrahiert. Nach Trocknen des verbliebenen Kristallisats erhielt man 22,1 g 1-(4-Carbomethoxyphenyl)-3-(5,6,7,8-tetrahydro-5,5,8,8-tetramethyl-2-naphthalenyl)-1,3-propandion, Schmp. 128°C.

3-(4-Carbomethoxyphenyl)-5-(5,6,7,8-tetrahydro-5,5,8,8-tetramethyl-2-naphthalenyl)pyrazol:

6,0 g (15 mmol) 1-(4-Carbomethoxyphenyl)-3-(5,6,7,8-tetrahydroxy-5,5,8,8-tetramethyl-2-naphthalenyl)-1,3-propandion und 1,2 g Hydrazinhydrat wurden in einem Gemisch aus 30 ml Tetrahydrofuran und 20 ml Methanol 4 h unter Rückfluß erhitzt. Man ließ abkühlen, goß auf Wasser und saugte den Niederschlag ab. Nach Umkristallisation aus Methanol wurden 3,8 g der Titelverbindung erhalten, Schmp. 155 - 156°C.

Beispiel 13

3-(4-Carbomethoxyphenyl)-5-(2,3-dihydro-1,1,2,3,3-pentamethyl-5(1H)-indenyl)pyrazol

Analog Beispiel 12 erhielt man aus 23 g (0,1 mol) 5-Acetyl-2,3-dihydro-1,1,2,3,3-pentamethyl-(1H)-inden 18,1 g 1-(4-Carbomethoxyphenyl)-3-(2,3-dihydro-1,1,2,3,3-pentamethyl-5(1 H)-indenyl)-1,3-propandion und aus 5 g (13 mmol) davon 1,5 g der Titelverbindung.

Beispiel 14

3-(4-Carbomethoxyphenyl)-5-(5,6,7,8-tetrahydro-3,8,8-trimethyl-2-naphthalenyl)pyrazol

Analog Beispiel 12 erhielt man aus 10 g (46 mmol) 7-Acetyl-1,2,3,4-tetrahydro-1,1,6-trimethylnaphthalin 4,8 g 1-(4-Carbomethoxyphenyl)-3-(5,6,7,8-tetrahydro-3,8,8-trimethyl-naphthalenyl)-1,3-propandion, Schmp. 91 - 92 °C, und aus 2,9 g (7,6 mmol) davon 2,8 g der Titelverbindung, Schmp. 92 -93 °C.

Beispiel 15

1-(4-Carboxyphenyl)-4-(5,6,7,8-tetrahydro-5,5,8,8-tetramethyl-2-naphthalenyl)pyrazol

Zu einer Lösung von 23,0 g (0,105 mol) Trimethylsulfoxoniumiodid in 100 ml trockenem Dimethylformamid wurden bei 0 - 10 °C 11,7 g (0,15 mol) Kaliumtert.-butanolat zugegeben. Man ließ auf Raumtemp. kommen und tropfte eine Lösung von 25,4 g (0,088 mol) 6-Dimethoxyacetyl-1,2,3,4-tetrahydro-1,1,4,4-tetramethylnaphthalin in 25 ml Dimethylformamid zu. Man ließ 1 h nachrühren und extrahierte mit Wasser/Ether. Die Etherphase wurde mit Wasser gewaschen, über Natriumsulfat getrocknet und eingeengt. Zurück blieben 23,5 g rohes 2-Dimethoxymethyl-2-(5,6,7,8-tetrahydro-5,5,8,8-tetramethyl-2-naphthalenyl)-

oxiran, das ohne zusätzliche Reinigung weiter eingesetzt wurde.

13,0 g (43 mmol) dieses Oxirans und 65 ml Eisessig wurden 5 h unter Rückfluß erhitzt. Dann gab man 6,5 g (43 mmol) 4-Hydrazinobenzoesäure zu und rührte unter Rückfluß bis zur vollständigen Umsetzung (dünnschichtchromatographisch kontrolliert). Man goß auf Wasser und extrahierte mit Ether. Die organische Phase wurde zweimal mit ges. Natriumhydrogencarbonatlösung gewaschen, über Natriumhydrogencarbonat/Natriumsulfat getrocknet und eingeengt. Der ölige Rückstand ergab nach Umkristallisationen aus n-Heptan und nochmals aus Isopropanol 2,1 g der Titelverbindung, Schmp. 229-232°C.

Beispiel 16

1-(4-Carboxyphenyl)-3-(5,6,7,8-tetrahydro-5,5,8,8-tetramethyl-2-naphthalenyl)-2-pyrazolin

2,8 g (10 mmol) 6-(3-Chlorpropionyl)-1,2,3,4-tetrahydro-1,1,4,4-tetramethylnaphthalin (Herstellung s. Beispiel 9) und 1,5 g (10 mmol) 4-Hydrazinobenzoesäure wurden in 40 ml Dimethylformamid bis zur vollständigen Umsetzung (dünnschichtchromatographisch kontrolliert) gerührt. Man goß auf Wasser, saugte das entstandene Kristallisat ab und wusch mit Wasser und Ethanol nach. Nach Trocknen verblieben 2,2 g der Titelverbindung, Schmp. 276 - 278°C.

Beispiel 17

1-(4-Carboxyphenyl)-3-(5,6,7,8-tetrahydro-5,5,8,8-tetramethyl-2-naphthalenyl)-Δ2-pyrazolin-5-on

3-Oxo-3-(5,6,7,8-tetrahydro-5,5,8,8-tetramethyl-2-naphthalenyl)propionsäureethylester:

15 g (0,23 mol) Zinkpulver und 1,5 g (8,3 mmol) Kupfer(II)-acetat-monohydrat wurden in 50 ml Eisessig 30 min. lang unter Eiskühlung gerührt. Danach verrührte man mit 50 ml trockenem Ether, saugte den Feststoff ab und wusch ihn 2x mit trockenem Ether und 1x mit trockenem Tetrahydrofuran. Zu einer Suspension des solchermaßen hergestellten Zink/Kupfer-Paares tropfte man eine Lösung von 21,6 g (0,1 mol) 5,6,7,8-Tetrahydro-5,5,8,8-tetramethyl-2-naphthaldehyd und 21,0 g (0,125 mol) Bromessigsäureethylester in 500 ml trockenem Tetrahydrofuran, wobei die Reaktion Rückflußtemperatur erreichte. Man ließ noch 75 min. unter Rückfluß rühren, säuerte nach dem Abkühlen mit 5 N Schwefelsäure an und filtrierte. Das Filtrat wurde mit Ether extrahiert, die organische Phase mit Wasser gewaschen, über Natriumsulfat getrocknet und eingeengt. Der Rückstand (27,8 g) wurde in 100 ml Aceton gelöst und bei 10 °C eine Lösung von 12,0 g (0,12 mol) Chrom(VI)-oxid in 36,8 ml Wasser und 12,9 ml konz. Schwefelsäure zugetropft. Man ließ 30 min. bei 10 °C nachrühren, goß danach auf Eis/Wasser und extrahierte mit Ether. Die organische Phase wurde 2x mit Wasser gewaschen, über Natriumsulfat getrocknet und eingeengt. Aus dem Rückstand erhielt man nach Destillation 9,0 g 3-Oxo-3-(5,6,7,8-tetrahydro-5,5,8,8-tetramethyl-2-naphthalenyl)-propionsäureethylester Sdp. 150 - 152°C/0,4 mbar.

1-(4-Carboxyphenyl)-3-(5,6,7,8-tetrahydro-5,5,8,8-tetramethyl-2-naphthalenyl)-Δ2-pyrazolin-5-on

1,5 g (5 mmol) 3-Oxo-3-(5,6,7,8-tetrahydro-5,5,8,8-tetramethyl-2-naphthalenyl)propionsäureethylester und 0,75 g (5 mmol) 4-Hydrazinobenzoesäure wurden in 40 ml Ethanol 4 h bei 40 °C gerührt. Der Ansatz wurde auf Wasser gegossen und mit Ether extrahiert. Die Etherphase wurde 2x mit Wasser gewaschen, über Natriumsulfat getrocknet und eingeengt. Aus dem festen Rückstand erhielt man nach Umkristallisation aus Methanol/Methylenchlorid 0,6 g der Titelverbindung, Schmp. 275 - 277 °C.

Beispiel 18

5-(4-Cyanophenyl)-3-(5,6,7,8-tetrahydro-3-methoxy-5,5,8,8-tetramethyl-2-naphthalenyl)isoxazol

5 g (20 mmol) 5,6,7,8-Tetrahydro-3-methoxy-5,5,8,8-tetramethyl-2-naphthaldehyd und 2,1 g (30 mmol) Hydroxylammoniumchlorid wurden in 32,5 ml trockenem Pyridin 2 h unter Rückfluß erhitzt. Danach ließ man abkühlen, goß auf Wasser, säuerte mit conc. Salzsäure an und saugte den ausgefallenen Niederschlag ab. Nach Trocknen verblieben 5,1 g 5,6,7,8-Tetrahydro-3-methoxy-5,5,8,8-tetramethyl -2-naphthaldoxim, Schmp. 182 - 183°C.

Zu einer Lösung von 5,0 g (19 mmol) 5,6,7,8-Tetrahydro-3-methoxy-5,5,8,8-tetramethyl-2-naphthaldoxim und 2,4 g (19 mmol) 4-Ethinylbenzonitril in 40 ml Methylenchlorid wurden bei 10 - 15°C 17,2 g einer 10

%igen wäßrigen Natriumhypochloritlösung zugetropft. Man ließ 1,5 h bei Raumtemp. nachrühren. Danach gab man das Reaktionsgemisch auf Wasser, trennte die Phasen und extrahierte die wäßrige Phase noch einmal mit Methylenchlorid. Die vereinigten organischen Extrakte wurden mit Natriumhydrogensulfitlösung gewaschen, über Natriumsulfat getrocknet und eingeengt. Der Rückstand wurde in heißem Methanol digeriert, man ließ abkühlen, saugte das Kristallisat ab und erhielt nach Trocknen 5,4 g der Titelverbindung, Schmp. 202 - 203°C.

Beispiele 19 - 24

Analog Beispiel 18 wurden die in der Tabelle I aufgeführten Isoxazole hergestellt.

Tabelle 1

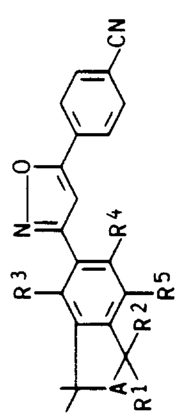

| Beispiel | A | $R^1$ | $R^2$ | $R^3$ | $R^4$ | $R^5$ | H-NMR (δ in ppm, Isoxazol-H) |
|---|---|---|---|---|---|---|---|
| 18 | $-CH_2CH_2-$ | $CH_3$ | $CH_3$ | H | $OCH_3$ | H | 7,16 |
| 19 | $-CH_2CH_2-$ | $CH_3$ | $CH_3$ | H | H | H | 6,9 |
| 20 | $-CH_2-CH_2-$ | $CH_3$ | $CH_3$ | H | $CH_3$ | H | 6,84 |
| 21 | $-CH_2-CH_2-$ | $CH_3$ | $CH_3$ | $OCH_3$ | $OCH_3$ | H | 6,86 |
| 22 | $-CH_2-CH_2$ | $CH_3$ | $CH_3$ | $OCH_3$ | H | $OCH_3$ | 7,18 |
| 23 | $-CH_2-CH_2$ | $CH_3$ | $CH_3$ | $O-n-C_3H_7$ | $CH_3$ | H | 6,82 |
| 24 | $-CH_2-CH_2$ | $CH_3$ | $CH_3$ | H | F | H | 7,08 |

Beispiel 25

5-(4-Formylphenyl)-3-(5,6,7,8-tetrahydro-5,5,8,8-tetramethyl-2-naphthalenyl)isoxazol

Zu einer Lösung von 1,5 g (4,2 mmol) 5-(4-Cyanophenyl)-3-(5,6,7,8-tetrahydro-5,5,8,8-tetramethyl-2-naphthalenyl)isoxazol (Beispiel 19) in 20 ml trockenem Ether tropfte man 8,8 ml einer 1 M Lösung von Diisobutylaluminiumhydrid in n-Hexan bei Raumtemp. unter Stickstoff zu und ließ 1 h nachrühren. Danach wurde mit 2 ml ges. Weinsäurelösung versetzt, etwas Magnesiumsulfat zugegeben, 15 min. gerührt, filtriert und das Filtrat eingeengt. Aus dem Rückstand erhielt man nach Umkristallisation aus Isopropanol 0,6 g der Titelverbindung, Schmp. 179 - 180°C.

Beispiel 26

5-[4-(Aminomethyl)phenyl)-3-(5,6,7,8-tetrahydro-5,5,8,8-tetramethyl-2-naphthalenyl)isoxazol

Zu einer Suspension von 0,28 g (7,4 mmol) Lithiumaluminiumhydrid in 30 ml trockenem Ether wurden 0,95 g (2,7 mmol) 5-(4-Cyanophenyl)-3-(5,6,7,8-tetrahydro-5,5,8,8-tetramethyl-2-naphthalenyl)isoxazol (Beispiel 19) portionsweise bei Raumtemp. zugegeben. Danach wurde 3 h unter Rückfluß erhitzt. Nach dem Abkühlen hydrolysierte man vorsichtig mit Wasser, trennte die Phasen, extrahierte die wäßrige Phase nochmals mit Ether, trocknete die vereinigten organischen Extrakte über Natriumsulfat und engte ein. Als Rückstand verblieben 0,9 g der Titelverbindung, Schmp. 138 - 143°C.

Beispiel 27

2-(4-Carbomethoxyphenyl)-4-(5,6,7,8-tetrahydro-5,5,8,8-tetramethyl-2-naphthalenyl)oxazol

15 g (65 mmol) 6-Acetyl-1,2,3,4-tetrahydro-1,1,4,4-tetramethylnaphthalin und 6,5 g (91 mmol) Hydroxylammoniumchlorid wurden in 100 ml Pyridin 1 h bei 80 °C erhitzt. Nach dem Abkühlen goß man auf Wasser und säuerte mit 2 N Salzsäure an. Das ausgefallene Kristallisat wurde abgesaugt, mit Wasser nachgewaschen und getrocknet. Man erhielt so 16 g 6-[(1-(Hydroximino)ethyl)]-1,2,3,4-tetrahydro-1,1,4,4-tetramethylnaphthalin. 5 g (20 mmol) davon und 5 g (20 mmol) Terephthalsäuremonomethylesterchlorid wurden zusammengegeben und 6 h bei 120 °C gerührt. Nach dem Abkühlen wurde die erstarrte Masse mit ca. 60 ml Methanol angelöst. Die überstehende Methanolphase wurde abdekantiert und der feste Rückstand aus Chloroform/Methanol zweimal umkristallisiert. Man erhielt so 2,5 g der Titelverbindung, Schmp. 207 - 208°C.

Beispiel 28

2-(4-Carbomethoxyphenyl)-5-(5,6,7,8-tetrahydro-5,5,8,8-tetramethyl-2-naphthalenyl)oxazol

6-Chloracetyl-1,2,3,4-tetrahydro-1,1,4,4-tetramethylnaphthalin:

Zu einer Lösung von 72 g (0,55 mol) wasserfreiem Aluminiumchlorid und 40,7 g (0,36 mol) Chloressigsäurechlorid in 270 ml trockenem Methylenchlorid wurde bei 0 - 5 °C eine Lösung von 68 g (0,36 mol) 1,2,3,4-Tetrahydro-1,1,4,4-tetramethylnaphthalin in 200 ml Methylenchlorid zugetropft. Danach ließ man über Nacht bei Raumtemperatur rühren. Man goß auf Eis/Wasser, extrahierte mit Methylenchlorid, wusch die organische Phase mehrmals mit Wasser, trocknete über Natriumsulfat und engte ein. Es verblieben 92,2 g 6-Chloracetyl-1,2,3,4-tetrahydro-1,1,4,4-tetramethylnaphthalin als Öl. Die Struktur wurde durch H-NMR-Spektroskopie abgesichert.

6-Azidoacetyl-1,2,3,4-tetrahydro-1,1,4,4-tetramethylnaphthalin:

Zu einer Lösung von 30 g (113 mmol) 6-Chloracetyl-1,2,3,4-tetrahydro-1,1,4,4-tetramethylnaphthalin in 120 ml Dimethylsulfoxid wurden portionsweise 8,9 g (136 mmol) Natriumazid eingetragen. Man ließ 1 h bei Raumtemp. nachrühren, goß danach auf Eis/Wasser, extrahierte mit Essigester, wusch die organische Phase mit Wasser, trocknete über Natriumsulfat und engte ein. Es verblieben 32,4 g rohes 6-Azidoacetyl-1,2,3,4-tetrahydro-1,1,4,4-tetramethylnaphthalin. Die Struktur wurde durch H-NMR-Spektroskopie abgesichert.

6-Aminoacetyl-1,2,3,4-tetrahydro-1,1,4,4-tetramethylnaphthalin-hydrochlorid:

16,2 g (63,5 mmol) 6-Aminoacetyl-1,2,3,4-tetrahydro-1,1,4,4-tetramethylnaphthalin wurden in 250 ml Methanol und 59 ml 2 N Salzsäure mit 2,9 g 10 %igem Palladium auf Aktivkohle bei Raumtemp. und Normaldruck hydriert. Das Reaktionsgemisch wurde über Celite filtriert und das Filtrat eingeengt. Der Rückstand wurde mit Ether und wenigen Tropfen Methanol verrührt und die ausgefallenen Kristalle abgesaugt. Nach Trocknen verblieben 5,8 g 6-Aminoacetyl-1,2,3,4-tetrahydro-1,1,4,4-tetramethylnaphthalin-hydrochlorid. Die Struktur wurde durch H-NMR-Spektroskopie abgesichert.

2-N-(4-Carbomethoxybenzoyl)amino-1-(5,6,7,8-tetrahydro-5,5,8,8-tetramethyl-2-naphthalenyl)ethanon:

Zu einer Lösung von 5,6 g (20 mmol) 6-Aminoacetyl-1,2,3,4-tetrahydro-1,1,4,4-tetramethylnaphthalin-hydrochlorid und 4,0 g (20 mmol) Terephthalsäuremonomethylesterchlorid in 40 ml Dimethylformamid wurden bei 0 bis 10°C 5 g Triethylamin zugetropft. Man ließ 15 min. nachrühren und dann auf Raumtemp. kommen. Das Reaktionsgemisch wurde auf Wasser gegossen und 3x mit Ether extrahiert. Man wusch die vereinigten organischen Extrakte mit Wasser, trocknete über Natriumsulfat und engte ein. Der Rückstand ergab nach Umkristallisation aus n-Heptan/Toluol 4,4 g 2-N-(4-Carbomethoxybenzoyl)amino-1-(5,6,7,8-tetrahydro-5,5,8,8-tetramethyl-2-naphthalenyl)ethanon, Schmp. 126 - 128°C.

2-(4-Carbomethoxyphenyl)-5-(5,6,7,8-tetrahydro-5,5,8,8-tetramethyl-2-naphthalenyl)oxazol:

3 g (7,4 mmol) 2-N-(4-Carbomethoxybenzoyl)amino-1-(5,6,7,8-tetrahydro5,5,8,8-tetramethyl-2-naphthalenyl)ethanon wurden in 20 ml konz. Schwefelsäure 20 min. bei Raumtemp. gerührt. Man goß auf Wasser, rührte 15 min. nach und saugte das Kristallisat ab. Nach Trocknen erhielt man 2,8 g der Titelverbindung, Schmp. 153 - 155°C.

Beispiel 29

3-(4-Carbethoxyphenyl)-5-(5,6,7,8-tetrahydro-5,5,8,8-tetramethyl-2-naphthalenyl)oxazolidin

(5,6,7,8-Tetrahydro-5,5,8,8-tetramethyl-2-naphthalenyl)oxiran:

Zu einer Lösung von 17 g (0,15 mol) Kalium-tert.-butanolat in 150 ml trockenem Dimethylsulfoxid wurden bei Raumtemp. 33 g (0,15 mol) Trimethylsulfoxoniumiodid zugegeben. Man ließ 0,5 h nachrühren und tropfte dann eine Lösung von 32,5 g (0,15 mol) 5,6,7,8-Tetrahydro-5,5,8,8-tetramethyl-2-naphthaldehyd in 100 ml Tetrahydrofuran zu. Man ließ 1 h nachrühren, goß danach auf Wasser und extrahierte mit Ether. Die organische Phase wurde mit Wasser gewaschen, über Magnesiumsulfat getrocknet und eingeengt. Es verblieben 31,3 g (5,6,7,8-Tetrahydro-5,5,8,8-tetramethyl-2-naphthalenyl)oxiran. Die Struktur wurde durch H-NMR-Spektroskopie abgesichert.

N-(4-Carbethoxyphenyl)-1-(5,6,7,8-tetrahydro-5,5,8,8-tetramethyl-2-naphthalenyl)-2-aminoethanol:

26 g (110 mmol) (5,6,7,8-Tetrahydro-5,5,8,8-tetramethyl-2-napthalenyl)oxiran, 15 g (90 mmol) 4-Aminobenzoesäureethylester, 135 g basisches Aluminiumoxid und 450 ml Toluol wurden 2 h unter Rückfluß erhitzt. Danach wurde vom Feststoff abfiltriert und das Filtrat eingeengt. Der Rückstand lieferte nach Umkristallisation aus n-Heptan mit wenig Essigester 11,0 g N-(4-Carbethoxyphenyl)-1-(5,6,7,8-tetrahydro-5,5,8,8-tetramethyl-2-naphthalenyl)-2-aminoethanol. Die Struktur wurde durch H-NMR-Spektroskopie abgesichert.

3-(4-Carbethoxyphenyl)-5-(5,6,7,8-tetrahydro-5,5,8,8-tetramethyl-2-naphthalenyl)oxazolidin:

1,58 g (4 mmol) N-(4-Carbethoxyphenyl)-1-(5,6,7,8-tetrahydro-5,5,8,8-tetramethyl-2-naphthalenyl)-2-aminoethanol wurden mit 20 ml 1 N Natriumhydrogenphosphitlösung und 2 ml (20 mmol) Formalinlösung in 20 ml Dioxan 1 h bei 60 °C gerührt. Dann goß man auf Wasser, extrahierte mit Ether, wusch die organische Phase mit 2 N Natronlauge und Wasser, trocknete über Magnesiumsulfat und engte ein. Nach Umkristallisation aus Methanol erhielt man 1,0 g der Titelverbindung, $R_F$ = 0,51 (Dünnschichtchromatograhie: Kieselgel; n-Heptan/Essigester 7 : 1).

Beispiel 30

2-(4-Carbomethoxyphenyl)-4-(5,6,7,8-tetrahydro-5,5,8,8-tetramethyl-2-naphthalenyl)thiazol:

130 g (1,7 mol) Thioessigsäure, 50 g (0,3 mol) 4-Cyanobenzoesäuremethylester und 12 ml Eisessig wurden 2 h bei 80°C gerührt. Man ließ abkühlen, versetzte mit 200 ml Isopropanol/Wasser (1 : 1), rührte kurze Zeit und saugte das gebildete Kristallisat ab. Man erhielt so 45,5 g 4-Carbomethoxybenzthiamid, Schmp. 188 - 190°C.

3,2 g (16,5 mol) des 4-Carbomethoxybenzthiamids und 4,2 g (16 mmol) 6-Chloracetyl-1,2,3,4-tetrahydro-1,1,4,4-tetramethylnaphthalins (Herstellung s. Beispiel 28) wurden in 40 ml Isopropanol unter Zusatz von 2 Tropfen Pyridin 1 h unter Rückfluß erhitzt. Nach dem Abkühlen wurde mit Wasser versetzt und das gebildete Kristallisat abgesaugt. Nach Umkristallisation aus Isopropanol/Spur Wasser erhielt man 3,0 g der Titelverbindung, Schmp. 138 - 140°C.

Beispiel 31

4-(4-Carbethoxyphenyl)-2-(5,6,7,8-tetrahydro-5,5,8,8-tetramethyl-2-naphthalenyl)imidazol

2,6 g (10 mmol) 6-Chloracetyl-1,2,3,4-tetrahydro-1,1,4,4-tetramethylnaphthalin (Herstellung s. Beispiel 28), 2,1 g (10 mmol) 4-Carbethoxybenzamidiniumchlorid und 5 g Kaliumcarbonat wurden in 100 ml Dimethylformamid 1 h unter Rückfluß erhitzt. Nach dem Abkühlen goß man auf Wasser, rührte 10 min. und saugte den gebildeten Niederschlag ab. Nach säulenchromatographischer Reinigung (Kieselgel; n-Heptan/Essigester 9 : 1) erhielt man 0,3 g der Titelverbindung, Schmp. 186 - 188°C.

Beispiel 32

5-(5,6,7,8-Tetrahydro-5,5,8,8-tetramethyl-2-naphthalenyl)-3-(4-Tolyl)-1,2,4-oxadiazol

8,0 g (30 mmol) 5,6,7,8-Tetrahydro-5,5,8,8-tetramethyl-2-naphthoesäurechlorid und 4,5 g (30 mmol) 4-Methylbenzamidoxim wurden in 250 ml Dioxan 1 h unter Rückfluß erhitzt. Dann wurde 1 ml Bortrifluoriddietherat zugegeben und 4 h nochmals unter Rückfluß erhitzt. Man ließ abkühlen, goß auf Eis/Wasser und extrahierte mit Methylenchlorid. Die organische Phase wurde mehrmals mit Wasser gewaschen, über Magnesiumsulfat getrocknet und eingeengt. Der ölige Rückstand wurde mittels Säulenchromatographie (Kieselgel; n-Heptan + 0,5 % Essigester) aufgereinigt, und das aus den ersten Fraktionen erhaltene Rohprodukt ergab nach Umkristallisation aus Methanol 3,2 g der Titelverbindung, Schmp. 106 - 109 °C.

Beispiel 33

5-(4-Carbomethoxyphenyl)-3-(5,6,7,8-tetrahydro-5,5,8,8-tetramethyl-2-naphthalenyl)-1,2,4-oxadiazol

Zu einer siedenden Lösung von 21,6 g (0,1 mol) 5,6,7,8-Tetrahydro-5,5,8,8-tetramethyl-2-naphthaldehyd in 25 ml Dimethylformamid wurden portionsweise 7,6 g (0,11 mol) Hydroxylammoniumchlorid so eingetragen, daß das Reaktionsgemisch am Sieden blieb. Man ließ 30 min. bei Rückflußtemp. nachrühren, verdünnte nach dem Abkühlen mit dem dreifachen Volumen Wasser und extrahierte mit Ether. Die organische Phase wurde mit Wasser mehrmals gewaschen, über Magnesiumsulfat getrocknet und eingeengt. Zurück blieben 17,2 g öliges 6-Cyano-1,2,3,4-tetrahydro-1,1,4,4-tetramethylnaphthalin. 15 g (70 mmol) dieses Öls, 4,8 g (35 mmol) Kaliumcarbonat und 4,9 g (70 mmol) Hydroxylammoniumchlorid wurden in 175 ml Ethanol und 35 ml Wasser 10 h unter Rückfluß erhitzt. Nach dem Abkühlen wurden Feststoffe abfiltriert und das Filtrat eingedampft. Der Rückstand wurde mit Methylenchlorid/Wasser extrahiert, die organische Phase wurde abgetrennt, über Magnesiumsulfat getrocknet und eingeengt. Es verblieben 17,2 g 5,6,7,8-Tetrahydro-5,5,8,8-tetramethylnaphthalin-2-carbonsäureamidoxim als feste Masse, die ohne zusätzliche Reinigung weiter umgesetzt wurde.

Nach dem in Beispiel 32 beschriebenen Verfahren erhielt man in ähnlicher Weise aus 6,5 g (30 mmol) Terephthalsäuremonomethylesterchlorid und 7,4 g (30 mmol) 5,6,7,8-Tetrahydro-5,5,8,8-tetramethylnaphthalin-2-carbonsäureamidoxim 4,1 g der Titelverbindung, Schmp. 166 - 168 °C (aus Pentan).

Beispiel 34

2-(4-Carbamoylphenyl)-5-(5,6,7,8-tetrahydro-5,5,8,8-tetramethyl-2-naphthalenyl)-1,3,4-oxadiazol

N-(4-Cyanobenzoyl)-N'-(5,6,7,8-tetrahydro-5,5,8,8-tetramethyl-2-naphthoyl)hydrazin:

23,2 g (0,1 mol) 5,6,7,8-Tetrahydro-5,5,8,8-tetramethylnaphthalin-2-carbonsäure und 18 g (0,15 mol) Thionylchlorid wurden in 75 ml Toluol erhitzt, bis sich kein Chlorwasserstoffgas mehr bildete. Danach engte man ein und gab mehrmals Toluol zu und engte wieder ein zur Entfernung von Thionylchloridresten. Der Rückstand wurde in 75 ml Isopropanol gelöst und bei -10 bis -15 °C zu einer Lösung von 7,4 g (0,23 mol) Hydrazin in 200 ml Isopropanol zugetropft. Man ließ 15 min. bei -10 °C nachrühren, gab 150 ml Wasser und 50 ml Ether zu und rührte nochmals kräftig durch. Nach Stehenlassen über Nacht wurde vom Feststoff abfiltriert und das Filtrat mit Wasser/Ether extrahiert. Die organische Phase wurde mit Wasser gewaschen, über Natriumsulfat getrocknet und eingeengt. 19,7 g (0,08 mol) des als Rückstand verbliebenen 5,6,7,8-Tetrahydro-5,5,8,8-tetramethylnaphthalin-2-carbonsäurehyd razids wurden in 100 ml trockenem Tetrahydrofuran gelöst. Dazu wurde bei 0 °C eine Lösung von 13,5 g (0,088 mol) 4-Cyanobenzoylchlorid in 100 ml Tetrahydrofuran getropft. Man ließ das Reaktionsgemisch unter Rühren während 45 min. auf Raumtemp. kommen. Man goß auf Wasser, filtrierte den ausgefallenen Feststoff ab, wusch mit Ethanol nach und erhielt nach Trocknen 16,4 g N-(4-Cyanobenzoyl)-N'-(5,6,7,8-tetrahydro-5,5,8,8-tetramethyl-2-naphthoyl)hydrazin, Schmp. 274 - 277°C.

2-(4-Carbamoylphenyl)-5-(5,6,7,8-tetrahydro-5,5,8,8-tetramethyl-2-naphthalenyl)-1,3,4-oxadiazol:

5,8 g (15,5 mmol) N-(4-Cyanobenzoyl-N'-(5,6,7,8-tetrahydro-5,5,8,8-tetramethyl-2-naphthalenyl)-hydrazin und 75 g Polyphosphorsäure wurden zusammengegeben und 6 h bei 100 °C gerührt. Danach goß man auf Eis/Wasser und saugte das ausgefallene Kristallisat ab. Das noch feuchte Kristallisat wurde aus Isopropanol umkristallisiert, und man erhielt so 3,3 g der Titelverbindung, Schmp. 272 - 274 °C.

Beispiel 35

5-(4-Carbomethoxyphenyl)-3-(5,6,7,8-tetrahydro-5,5,8,8-tetramethyl-2-naphthalenyl)pyridazin

8 g (18 mmol) 3-(4-Carbomethoxyphenyl)-4-dimethoxy-1-(5,6,7,8-tetrahydro5,5,8,8-tetramethyl-2-naphthalenyl)-1-butanon (Herstellung s. Beispiel 1) und 3,3 g Hydrazinhydrat wurden in 150 ml Essigsäure 1 h unter Rückfluß erhitzt. Nach dem Abkühlen gab man auf Wasser, neutralisierte mit 2 N Natronlauge auf pH 5 - 6, saugte den gebildeten Niederschlag ab und trocknete ihn. Nach Umkristallisation aus Isopropanol/n-Heptan erhielt man 1,5 g der Titelverbindung, H-NMR ($CDCl_3$) : $\delta$ = 8,02 und 9,39 ppm (jeweils d, 2 H, Pyradizin-H).

Beispiel 36

3-(4-Carbomethoxyphenyl)-6-(5,6,7,8-tetrahydro-5,5,8,8-tetramethyl-2-naphthalenyl)pyridazin

Zu einer Lösung von 4,0 g (10 mmol) 1-(4-Carbomethoxyphenyl)-4-(5,6,7,8-tetrahydro-5,5,8,8-tetramethyl-2-naphthalenyl)-1,4-butandion (Herstellung s. Beispiel 9) in 50 ml Ethanol wurden 0,3 g (10 mmol) Hydrazin (100 %) bei Raumtemp. zugetropft. Man erhitzte anschließend 20 min unter Rückfluß. Das Reaktionsgemisch wurde nach dem Abkühlen in Wasser eingerührt, die gelben Kristalle wurden abgesaugt und im Stickstoffstrom getrocknet. Zur Aroamtisierung von nicht oxidiertem Dihydropyridazin wurde das Rohprodukt mit Periodsäure in Ethanol gerührt, bis nach dünnschichthromatographischer Kontrolle hauptsächlich ein einheitliches Produkt vorlag. Man verdünnte dann mit Wasser, machte mit 2 N Natronlauge basisch und extrahierte mit Methylenchlorid. Die organische Phase wurde mit Wasser gewaschen, über Magnesiumsulfat getrocknet und eingeengt. Es verblieben 3,1 g der Titelverbindung als Öl, H-NMR ($CF_3COOH$) : = 8,82 und 8,88 ppm (jeweils d, 2 H, Pyridazin-H).

Beispiel 37

4-(4-Carbobutoxyphenyl)-2-hydroxy-6-(5,6,7,8-tetrahydro-5,5,8,8-tetramethyl-2-naphthalenyl)pyrimidin

21 g (0,1 mol) 6-Ethinyl-1,2,3,4-tetrahydro-1,1,4,4-tetramethylnaphthalin, 16 g (0,1 mol) 4-Formylbenzoesäuremethylester, 9 g (0,15 mol) Harnstoff und 15 ml konz. Schwefelsäure wurden in 50 ml n-Butanol 4 h unter Rückfluß erhitzt. Nach dem Abkühlen goß man auf 500 ml Wasser, versetzte mit 100 ml Ether und ließ 5 min. rühren. Es fiel ein Feststoff zwischen den Phasen aus. Im Schütteltrichter wurde die wäßrige Phase abgetrennt, der Rest nochmals mit Wasser gewaschen und dann mit 200 ml Petrolether durchgeschüttelt. Der Niederschlag wurde abgesaugt und mit Petrolether nachgewaschen. Man erhielt so 6,5 g der Titelverbindung, Schmp. 267 - 277°C.

Beispiel 38

1-(4-Carboxyphenyl)-2-mercapto-4-(5,6,7,8-tetrahydro-5,5,8,8-tetramethyl-2-naphthalenyl)imidazol

2-N-(4-Carboxyphenyl)amino-1-(5,6,7,8-tetrahydro-5,5,8,8-tetramethyl-2-naphthalenyl)ethanon

Zu einer Lösung von 25 g (0,186 mol) 4-Aminobenzoesäureethylester in 70 ml Dimethylformamid wurden unter Kühlung zunächst 40 g (0,4 mol) Triethylamin zugegeben, und dann ebenfalls unter Kühlung eine Lösung von 41 g (0,4 mol) 6-Chloracetyl-1,2,3,4-tetrahydro-1,1,4,4-tetramethylnaphthalin (Herstellung s. Beispiel 28) zugetropft. Man ließ anschließend 3 Stunden bei Raumtemp. rühren. Das Reaktionsgemisch wurde dann auf Wasser gegeben, 30 min. im Eisbad gerührt, der entstandene Niederschlag abgesaugt, mit Wasser gewaschen und getrocknet. Nach Umkristallisation aus Ethanol erhielt man 22,6 g 2-N-(4-Carboxyphenyl)amino-1-(5,6,7,8-tetrahydro-5,5,8,8-tetramethyl-2-naphthalenyl)ethanon, Schmp. 168 - 169°C.

1-(4-Carboxyphenyl)-2-mercapto-4-(5,6,7,8-tetrahydro-5,5,8,8-tetramethyl-2-naphthalenyl)imidazol

3,6 g (10 mmol) 2-N-(4-Carboxyphenyl)amino-1-(5,6,7,8-tetrahydro-5,5,8,8-tetramethyl-2-naphthalenyl)-ethanon wurden in 40 ml 1 N Salzsäure suspendiert. Man gab 2 g Kaliumthiocyanat zu und erhitzte auf Rückflußtemperatur. Bald danach bildete sich eine zweite Phase; man ließ auf 60°C abkühlen, gab 30 ml Isopropanol zu und erhitzte 9 h unter Rückfluß. Nach dem Abkühlen wurde der Feststoff abfiltriert und mit Wasser gewaschen. Nach Umkristallisation aus Methanol/Aceton erhielt man 1,3 g der Titelverbindung, Schmp. 170 - 172 °C.

Beispiel 39 - 61

Die Beispiele 39 - 61 der nachstehenden Tabelle 2 wurden nach folgender allgemeiner Vorschrift erhalten:

6 mmol Ester oder Nitril wurden in einem Gemisch von 16 ml 10 N Natronlauge und 22 ml Ethanol 2 h unter Rückfluß erhitzt. Nach dem Abkühlen gab man auf Wasser, säuerte mit konz. Schwefelsäure an, saugte den gebildeten Niederschlag ab und trocknete ihn. Gegebenenfalls wurde dann noch aus einem geeigneten Lösungsmittel umkristallisiert.

Tabelle 2

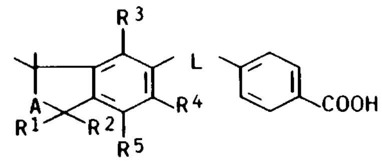

| Beispiel | A | $R^1$ | $R^2$ | $R^3$ | $R^4$ | $R^5$ | L | aus $R^6$ = | Schmp. (°C) |
|---|---|---|---|---|---|---|---|---|---|
| 39 | $-CH_2CH_2-$ | $CH_3$ | $CH_3$ | H | H | H | 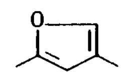 | $-COOCH_3$ | 215 - 217 |
| 40 | $-CH_2-CH_2-$ | $CH_3$ | $CH_3$ | H | H | H | 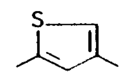 | $-COOCH_3$ | 235 - 237 |
| 41 | $-CH_2-CH_2-$ | $CH_3$ | $CH_3$ | H | H | H | 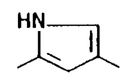 | $-COOCH_3$ | 266 - 267 |
| 42 | $-CH_2-CH_2-$ | $CH_3$ | $CH_3$ | H | H | H | 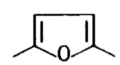 | $-COOCH_3$ | 261 - 262 |
| 43 | $-CH_2-CH_2-$ | $CH_3$ | $CH_3$ | H | H | H | 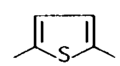 | $-COOCH_3$ | 281 - 285 |
| 44 | $-CH_2-CH_2-$ | $CH_3$ | $CH_3$ | H | H | H | 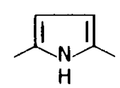 | $-COOCH_3$ | 269 - 271 |

Tabelle 2 (Fortsetzung)

| Beispiel | A | $R^1$ | $R^2$ | $R^3$ | $R^4$ | $R^5$ | L | aus $R^6$ = | Schmp. (°C) |
|---|---|---|---|---|---|---|---|---|---|
| 45 | $-CH_2-CH_2-$ | $CH_3$ | $CH_3$ | H | H | H | 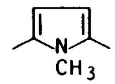 | $-COOCH_3$ | 217 - 219 |
| 46 | $-CH_2CH_2$ | $CH_3$ | $CH_3$ | H | H | H | 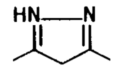 | $-COOCH_3$ | 289 - 290 |
| 47 | $-CH_2CH_2-$ | H | H | H | $CH_3$ | H | 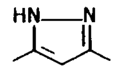 | $-COOCH_3$ | 300 |
| 48 | $-CH_3-\underset{CH_3}{\overset{CH_3}{CH}}-$ | $CH_3$ | $CH_3$ | H | H | H | 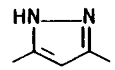 | $-COOCH_3$ | 300 |
| 49 | $-CH_2CH_2-$ | $CH_3$ | $CH_3$ | H | H | H | 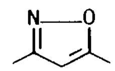 | $-CN$ | 167 - 168 |
| 50 | $-CH_2CH_2-$ | $CH_3$ | $CH_3$ | H | $OCH_3$ | H | 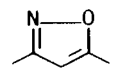 | $-CN$ | 228 - 229 |
| 51 | $-CH_2CH_2-$ | $CH_3$ | $CH_3$ | H | $CH_3$ | H | 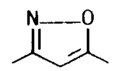 | $-CN$ | 237 - 238 |
| 52 | $-CH_2CH_2-$ | $CH_3$ | $CH_3$ | H | F | H | 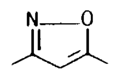 | $-CN$ | 250 |

Tabelle 2 (Fortsetzung)

| Beispiel | A | $R^1$ | $R^2$ | $R^3$ | $R^4$ | $R^5$ | L | aus $R^6$ = | Schmp. (°C) |
|---|---|---|---|---|---|---|---|---|---|
| 53 | $-CH_2CH_2-$ | $CH_3$ | $CH_3$ | $OCH_3$ | H | $OCH_3$ | 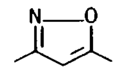 | -CN | 208 - 210 |
| 54 | $-CH_2CH_2-$ | $CH_3$ | $CH_3$ | $OCH_3$ | $OCH_3$ | H | 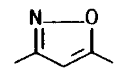 | -CN | 250 - 252 |
| 55 | $-CH_2CH_2-$ | $CH_3$ | $CH_3$ | $O-n-C_3H_7$ | $CH_3$ | H | 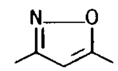 | -CN | 258 - 265 |
| 56 | $-CH_2CH_2-$ | $CH_3$ | $CH_3$ | H | H | H | 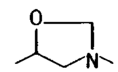 | $-COOC_2H_5$ | 258 - 265 |
| 57 | $-CH_2CH_2-$ | $CH_3$ | $CH_3$ | H | H | H | 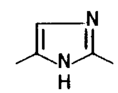 | $-COOCH_3$ | |
| 58 | $-CH_2CH_2-$ | $CH_3$ | $CH_3$ | H | H | H | 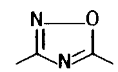 | $-COOCH_3$ | 263 - 265 |
| 59 | $-CH_2CH_2-$ | $CH_3$ | $CH_3$ | H | H | H | 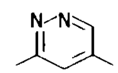 | $-COOCH_3$ | 300 |
| 60 | $-CH_2CH_2-$ | $CH_3$ | $CH_3$ | H | H | H | 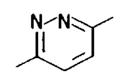 | $-COOCH_3$ | 320 - 324 |

Tabelle 2 (Fortsetzung)

| Beispiel | A | $R^1$ | $R^2$ | $R^3$ | $R^4$ | $R^5$ | L | aus $R^6$ = | Schmp. (°C) |
|---|---|---|---|---|---|---|---|---|---|
| 61 | $-CH_2CH_2-$ | $CH_3$ | $CH_3$ | H | H | H | 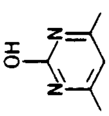 | $-COOC_4H_9$ | 320 |

Beispiele 62 - 64

Analog Beispiel 17 wurden die in der Tabelle 3 aufgeführten Pyrazolinone hergestellt. Bei schlechter Löslichkeit erhitzte man entweder unter Rückfluß oder katalysierte die Reaktion mit 0,05 Moläquivalenten Trifluoressigsäure. Statt der Extraktion mit Ether wurde das Reaktionsgemisch eingeengt und säulenchromatographisch (Kieselgel; n-Hexan/Essigester 95:5) vorgereinigt. Anschließend wurde aus Ether/n-Hexan oder Essigester/n-Hexan umkristallisiert.

Tabelle 3

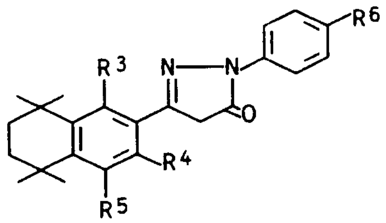

A

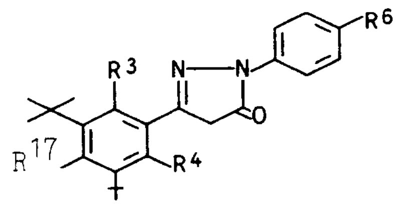

B

| Beispiel | Formel | $R^3$ | $R^4$ | $R^5$ | $R^{17}$ | $R^6$ | Schmp. (°C) |
|---|---|---|---|---|---|---|---|
| 62 | A | H | H | H | - | $-SO_2-CH_3$ | 246 - 248 |
| 63 | B | H | H | - | OH | $-SO_2-CH_3$ | 246 - 248 |
| 64 | B | H | H | - | OH | $-COOC_2H_5$ | 202 - 203 |
| 65 | A | H | H | H | - | $-SO_2-C_2H_5$ | 161 - 165 |
| 66 | A | H | H | H | - | $-SO_2-i-C_3H_7$ | 187 - 189 |

| Beispiel | |
|---|---|
| 67 | 1-(4-Methansulfonylphenyl)-4-(5,6,7,8-tetrahydro-5,5,8,8-tetramethyl-2-naphthalenyl)pyrazol, Schmp. 239-241°C, hergestellt analog Bsp. 15 |
| 68 | 1-(4-Methansulfonylphenyl)-3-(5,6,7,8-tetrahydro-5,5,8,8-tetramethyl-2-naphthalenyl)pyrazolin, Schmp. 234-236°C, hergestellt analog Bsp. 16 |
| 69 | 5-(4-Carboxyphenyl)-3-(5,6,7,8-tetrahydro-7-hydroxy-5,5,8,8-tetramethyl-2-naphthalenyl)isoxazol, Schmp. 289-291°C, hergestellt analog Bsp. 18 |

| Beispiel | |
|---|---|
| 70 | 2-(4-Ethansulfonylphenyl)-5-(5,6,7,8-tetrahydro-5,5,8,8-tetramethyl-2-naphthalenyl)pyrrol, Schmp. 254-257°C, hergestellt analog Bsp. 10 |
| 71 | 2-(4-Ethansulfonylphenyl)-5-(5,6,7,8-tetrahydro-5,5,8,8-tetramethyl-2-naphthalenyl)furan, Schmp. 239-241°C, hergestellt analog Bsp. 1 |
| 72 | 2-(4-Ethansulfonylphenyl)-5-(5,6,7,8-tetrahydro-5,5,8,8-tetramethyl-2-naphthalenyl)thiophen, Schmp. 183-185°C, hergestellt analog Bsp. 9 |

## Patentansprüche

1. Verbindungen der allgemeine Formel I

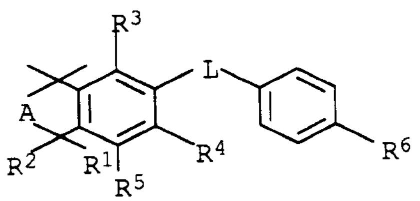

I,

worin:

$R^1$ und $R^2$ Wasserstoff oder Methyl darstellen,

A einen gegebenenfalls mit einer Methyl, Hydroxyl- oder Oxogruppe substituierten Ethylen- oder Methylenrest darstellt,

L einen gesättigten oder ungesättigten 5- oder 6-gliedrigen Heterocyclus mit 1 bis 3 Heteroatomen aus der Gruppe N, O und S, wobei das 2. und 3. Heteroatom ein Stickstoffatom ist, darstellt, der durch eine Hydroxyl-, Mercapto-, $C_{1-6}$-Alkyl oder $C_{1-4}$-Alkanoylgruppe und im Fall des Pyrazol-1,3-diyls sowie des Δ2-Pyrazolin-1,3-diyls in 5-Stellung auch durch $C_{1-4}$-Alkoxycarbonyl substituiert sein kann,

$R^3$ Wasserstoff, eine Hydroxy- oder eine $C_{1-6}$-Alkoxygruppeist,

$R^4$ für Wasserstoff, einen $C_{1-4}$-Alkylrest, ein Halogenatom oder eine Methoxygruppe steht,

$R^5$ Wasserstoff oder eine Methoxygruppe oder eine t-Butylgruppe ist und

$R^6$ Wasserstoff, eine Methyl- oder Nitrilgruppe, eine $C_{2-10}$-Ketalgruppe oder die Reste $-CHR^7-OR^8$, $-CHR^7-NR^9R^{10}$, $-COR^{11}$,
$-SR^{12}$ ,

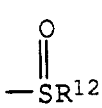 oder 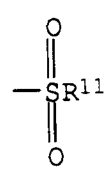

bedeutet, worin

$R^7$ Wasserstoff oder eine $C_{1-4}$-Alkylgruppe

$R^8$ Wasserstoff, eine $C_{1-4}$-Alkyl, $C_{1-20}$-Alkanoyl-, oder eine gegebenenfalls durch eine

Methoxy-, Nitro- oder Methylgruppe oder Chlor substituierte Benzoylgruppe,

$R^9$ und $R^{10}$ Wasserstoffatome, $C_{1-4}$-Alkyl, $C_{1-6}$-Alkanoyl- oder gegebenenfalls wie bei $R^8$ substituierte Benzoylgruppen oder zusammen mit dem Stickstoffatom, an das sie gebunden sind, einen gesättigten, 5- oder 6-gliedrigen heterocyclischen Rest, der Sauerstoff als 2. Heteroatom enthalten kann,

$R^{11}$ ein Wasserstoffatom, eine $C_{1-4}$-Alkylgruppe oder die Reste -$OR^{13}$ oder -$NR^{14}R^{15}$ mit $R^{13}$ in der Bedeutung von Wasserstoff, einer gegebenenfalls durch Hydroxylgruppen substituierten $C_{1-8}$-Alkylgruppe, einer gegebenenfalls durch ein Chlor- oder Bromatom oder eine Methyl-, Methoxy- oder Nitrogruppe substituierten Aryl- oder gegebenenfalls im Arylteil substituierten Aralkylgruppe, und mit $R^{14}$ und $R^{15}$ in der Bedeutung von Wasserstoffatomen, gegebenenfalls durch eine Hydroxygruppe substituierten $C_{1-6}$-Alkylgruppen, gegebenenfalls wie bei $R^{13}$ substituierten Aralkyl- oder Arylgruppen, oder $R^{14}$ und $R^{15}$ zusammen mit dem Stickstoffatom, an das sie gebunden sind, in der Bedeutung eines wie oben für $R^9$ und $R^{10}$ definierten heterocyclischen Restes

darstellen, und

$R^{12}$ eine $C_{1-4}$-Alkylgruppe bedeutet.

**2.** Verbindungen der allgemeinen Formel I nach Anspruch 1, wobei $R^6$ eine Nitrilgruppe, eine $C_{2-10}$-Ketalgruppe oder einen der Reste -$CHR^7$-$OR^8$, -$CHR^7$-$NR^9R^{10}$, -$COR^{11}$, -$SR^{12}$, -$S(O)R^{12}$ oder -$S(O)_2R^{11}$ bedeutet.

**3.** Verbindungen der allgemeinen Formel I nach Anspruch 1, wobei $R^6$ eine $C_{2-10}$-Ketalgruppe oder einen der Reste -$COR^{11}$, -$SR^{12}$, -$S(O)R^{12}$ oder -$SO_2R^{11}$ bedeutet.

**4.** Verbindungen der allgemeinen Formel I nach Anspruch 1, wobei $R^6$ einen der Reste -$COR^{11}$ oder $SO_2R^{11}$ bedeutet.

**5.** Verbindungen der allgemeinen Formel I nach Anspruch 1, wobei L Δ2-Pyrazolin-5-on-1,3-diyl darstellt.

**6.** Verbindungen der allgemeinen Formel I nach Anspruch 1, wobei L Pyrazol-1,3-diyl oder Δ2-Pyrazolin-1,3-diyl darstellt und diese Ringe jeweils in 5-Stellung durch Hydroxyl, $C_{1-3}$-Alkyl oder $C_{1-4}$-Alkoxycarbonyl substituiert sein können.

**7.** Verbindungen der allgemeinen Formel I nach Anspruch 1, wobei L einen Furan-, Thiophen- oder Pyrrolring und $R^6$ einen der Reste -$COR^{11}$ und -$SO_2R^{11}$ darstellt.

**8.** Verbindungen der allgemeinen Formel I nach Anspruch 1, wobei L Δ2-Pyrazolin-5-on-1,3-diyl und $R^6$ einen der Reste -$COR^{11}$ und -$SO_2R^{11}$ darstellt.

**9.** Verbindungen der allgemeinen Formel I nach Anspruch 1, wobei L Pyrazol-1,3-diyl oder Δ2-Pyrazolin-1,3-diyl darstellt und diese Ringe jeweils in 5-Stellung durch Hydroxyl, $C_{1-3}$-Alkyl oder $C_{1-4}$-Alkoxycarbonyl substituiert sein können und wobei $R^6$ einen der Reste -$COR^{11}$ und -$SO_2R^{11}$ darstellt.

**10.** Verbindungen der allgemeinen Formel I nach Anspruch 1, wobei L Furan-, Thiophen- oder Pyrrol-2,4- oder -2,5-diyl und $R^6$ die Carboxylgruppe darstellt.

**11.** 2-[(4-Methylsulfonyl)phenyl]-5-(5,6,7,8-tetrahydro-5,5,8,8-tetramethylnaphthalen-2-yl)pyrazolin-3-on.

**12.** Verbindungen der allgemeinen Formel Ia,

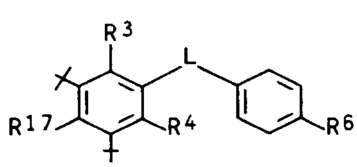

Ia

worin

$R^3$, $R^4$, $R^6$ und L die in Anspruch 1 genannten Bedeutungen haben und

$R^{17}$ = H oder OH ist,

sowie deren physiologisch verträgliche Salze.

**13.** 2-[(4-Methylsulfonyl)phenyl]-5-[(3,5-di-tert.butyl-4-hydroxy)phenyl]pyrazolin-3-on.

**14.** Verfahren zur Herstellung von Verbindungen der allgemeinen Formeln I nach Anspruch 1 durch folgende an sich bekannten Umsetzungen:

a) Umsetzung einer 1,3-Diketoverbindung der Formel II

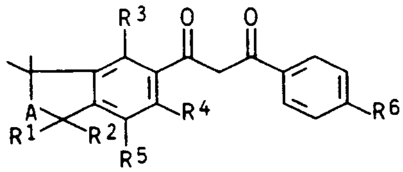

II,

worin

A und $R^1$ bis $R^6$ die in den Ansprüchen 1 bis 10 angegebenen Bedeutungen haben, mit Hydrazin, Hydroxylamin oder Harnstoff zu dem entsprechenden Pyrazol, Isoxazol oder 2-Hydroxypyrimidin;

b) Umsetzung einer 1,4-Diketoverbindung bzw. deren Ketal der Formeln III oder IV

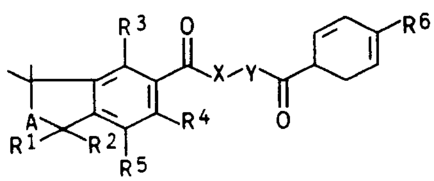

III,

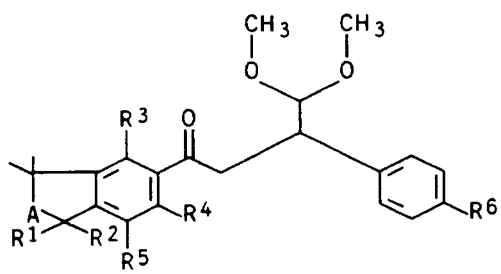

IV,

worin

A und $R^1$ bis $R^6$ die in den Ansprüchen 1 bis 10 angegebenen Bedeutungen haben, und

X und Y in Formel III für $-CH_2-$ oder -NH- stehen, mit dehydratisierenden Mitteln, ohne oder zusammen mit Sulfiden oder Schwefelwasserstoff, Ammoniak oder Ammoniumsalzen, prim. Aminen oder Hydrazin zu dem entsprechenden Furan, Thiophen, Pyrrol, Pyridazin, Oxazol oder 1,3,4-Oxadiazol;

d) 1,3-dipolare Cycloaddition eines Nitriloxids der Formel IX

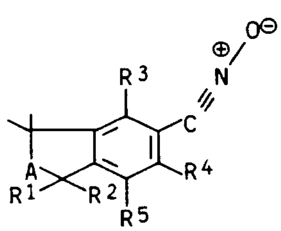

IX,

worin

A und $R^1$ bis $R^5$ die in den Ansprüchen 1 bis 10 angegebenen Bedeutungen haben, an ein Monoarylacetylen der Formel X

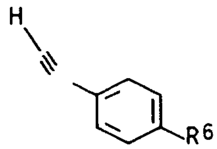

X,

worin

$R^6$ die im Anspruch 1 angegebenen Bedeutungen hat, zu Isoxazolen der Formel I.

**15.** Arzneimittel zur topischen Anwendung, das neben üblichen galenischen Hilfsstoffen 0,0001 bis 1 Gew.% einer Verbindung der allgemeinen Formeln I und Ia nach einem der Ansprüche 1 bis 12 als Wirkstoff enthält.

**16.** Arzneimittel zur systemischen Anwendung, das neben üblichen galenischen Hilfsstoffen pro Einzeldosis 0,1 bis 50 mg einer Verbindung der allgemeinen Formeln I und Ia nach einem der Ansprüche 1 bis 12 als Wirkstoff enthält.

**17.** Arzneimittel nach Anspruch 15 zur Behandlung von Akne oder Psoriasis oder anderen mit pathologisch veränderter Verhornung einhergehenden dermatologischen Erkrankungen sowie von Ekzemen, Warzen und Vitiligo.

**18.** Arzneimittel nach Anspruch 15 zur Behandlung von durch UV-Licht oder iatrogen bedingten Schädigungen der Haut.

**19.** Arzneimittel nach Anspruch 15 oder 16 zur Behandlung von Präkanzerosen und Tumoren.

**20.** Arzneimittel nach Anspruch 15 oder 16 zur Behandlung von rheumatischen und arthritischen Erkrankungen.

**21.** Arzneimittel mach Anspruch 15 oder 16 zur Behandlung von trockenen Augen und anderen Corneopathien.

**22.** Kosmetikum zur topischen Anwendung, das neben üblichen kosmetischen Hilfsstoffen 0,0001 bis 1 Gew.% einer Verbindung der allgemeinen Formeln I und Ia nach einem der Ansprüche 1 bis 12 als Wirkstoff enthält.

**23.** Kosmetikum nach Anspruch 15 zur Behandlung von Akne oder Psoriasis oder anderen mit pathologisch veränderter Verhornung einhergehenden dermatologischen Erkrankungen sowie von Ekzemen, Warzen und Vitiligo.

**24.** Kosmetikum nach Anspruch 15 zur Behandlung von durch UV-Licht oder iatrogen bedingten Schädigungen der Haut.

## Claims

**1.** A compound of the formula I

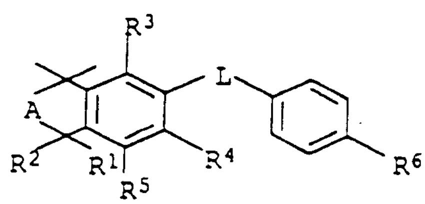

I

where $R^1$ and $R^2$ are each hydrogen or methyl,
A is an ethylene or methylene radical which is unsubstituted or substituted by methyl, hydroxyl or oxo,
L is a saturated or unsaturated 5-membered or 6-membered heterocyclic structure having from 1 to 3 heteroatoms from the group consisting of N, O and S, the second and third heteroatoms being a nitrogen atom, which may be substituted by hydroxyl, mercapto, $C_1$-$C_6$-alkyl or $C_1$-$C_4$-alkanoyl and, in the case of pyrazole-1,3-diyl and of $\Delta^2$-pyrazoline-1,3-diyl, also by $C_1$-$C_4$-alkoxycarbonyl in the 5-position,
$R^3$ is hydrogen or a hydroxyl or $C_1$-$C_6$-alkoxy group,
$R^4$ is hydrogen, $C_1$-$C_4$-alkyl, halogen or methoxy,
$R^5$ is hydrogen or methoxy or tert-butyl,
$R^6$ is hydrogen, methyl, nitrile or a $C_2$-$C_{10}$-ketal group or the radical $-CHR^7OR^8$, $-CHR^7-NR^9R^{10}$, $-COR^{11}$, $-SR^{12}$,
$—SR^{12}$ ,

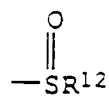
or
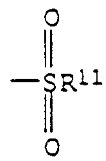

in which
$R^7$ is hydrogen or $C_1$-$C_4$-alkyl,
$R^8$ is hydrogen, $C_1$-$C_4$-alkyl or $C_1$-$C_{20}$-alkanoyl or is benzoyl which is unsubstituted or substituted by methoxy, nitro, methyl or chlorine,
$R^9$ and $R^{10}$ are each hydrogen, $C_1$-$C_4$-alkyl or $C_1$-$C_6$-alkanoyl or are each benzoyl which is unsubstituted or substituted as for $R^8$, or $R^9$ and $R^{10}$, together with the nitrogen atom to which they are bonded, form a saturated, 5-membered or 6-membered heterocyclic radical which may contain oxygen as the second heteroatom,
$R^{11}$ is hydrogen, $C_1$-$C_4$-alkyl, $-OR^{13}$ or $-NR^{14}R^{15}$, where $R^{13}$ is hydrogen, unsubstituted or hydroxyl-substituted $C_1$-$C_8$-alkyl, aryl or aralkyl which is unsubstituted or substituted by chlorine, bromine, methyl, methoxy or nitro, substitution in the case of the aralkyl group being in the aryl moiety, and where $R^{14}$ and $R^{15}$ are each hydrogen, unsubstituted or hydroxyl-substituted $C_1$-$C_6$-alkyl or an aralkyl or aryl group which is unsubstituted or substituted as for $R^{13}$, or $R^{14}$ and $R^{15}$, together with the nitrogen atom to which they are bonded, form a heterocyclic radical as defined above for $R^9$ and $R^{10}$, and
$R^{12}$ is $C_1$-$C_4$-alkyl.

**2.** A compound of the formula I as claimed in claim 1, wherein $R^6$ is nitrile, a $C_2$-$C_{10}$-ketal group or one of the radicals $-CHR^7-OR^8$, $-CHR^7-NR^9R^{10}$, $-COR^{11}$, $-SR^{12}$, $-S(O)R^{12}$ or $-S(O)_2R^{11}$.

**3.** A compound of the formula I as claimed in claim 1, wherein $R^6$ is a $C_2$-$C_{10}$-ketal group or one of the radicals $-COR^{11}$, $-SR^{12}$, $-S(O)R^{12}$ or $-SO_2R^{11}$.

**4.** A compound of the formula I as claimed in claim 1, wherein $R^6$ is one of the radicals $-COR^{11}$ or $SO_2R^{11}$.

**5.** A compound of the formula I as claimed in claim 1, wherein L is $\Delta^2$-pyrazolin-5-one-1,3-diyl.

**6.** A compound of the formula I as claimed in claim 1, wherein L is pyrazole-1,3-diyl or $\Delta^2$-pyrazoline-1,3-diyl and each of these rings may be substituted in the 5-position by hydroxyl, $C_1$-$C_3$-alkyl or $C_1$-$C_4$-alkoxycarbonyl.

**7.** A compound of the formula I as claimed in claim 1, wherein L is a furan, thiophene or pyrrole ring and $R^6$ is one of the radicals $-COR^{11}$ and $-SO_2R^{11}$.

**8.** A compound of the formula I as claimed in claim 1, wherein L is $\Delta^2$-pyrazolin-5-one-1,3-diyl and $R^6$ is one of the radicals $-COR^{11}$ and $-SO_2R^{11}$.

**9.** A compound of the formula I as claimed in claim 1, wherein L is pyrazole-1,3-diyl or $\Delta^2$-pyrazoline-1,3-diyl and each of these rings may be substituted in the 5-position by hydroxyl, $C_1$-$C_3$-alkyl or $C_1$-$C_4$-alkoxycarbonyl and $R^6$ is one of the radicals $-COR^{11}$ and $-SO_2R^{11}$.

**10.** A compound of the formula I as claimed in claim 1, wherein L is furan-, thiophene- or pyrrole-2,4- or -2,5-diyl and $R^6$ is carboxyl.

**11.** 2-[(4-Methylsulfonyl)-phenyl]-5-(5,6,7,8-tetrahydro-5,5,8,8-tetramethylnaphthalen-2-yl)-pyrazolin-3-one.

**12.** A compound of the formula Ia

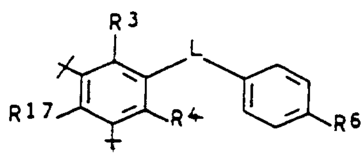

Ia

where $R^3$, $R^4$, $R^6$ and L have the meanings stated in claim 1, and
$R^{17}$ is H or OH,
or a physiologically tolerated salt thereof.

**13.** 2-[(4-Methylsulfonyl)-phenyl)-5-[(3,5-di-tert-butyl-4-hydroxy)-phenyl)-pyrazolin-3-one.

**14.** A process for the preparation of a compound of the formula I as claimed in claim 1 by the following known reactions:

a) Reaction of a 1,3-diketo compound of the formula II

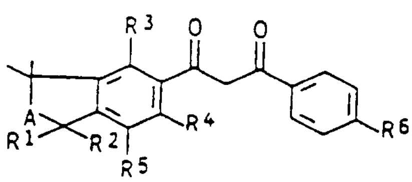

II

where A and $R^1$ to $R^6$ have the meanings stated in claims 1 to 10, with hydrazine, hydroxylamine or urea to give the corresponding pyrazole, isoxazole or 2-hydroxypyrimidine;

b) Reaction of a 1,4-diketo compound or of its ketal of the formula III or IV

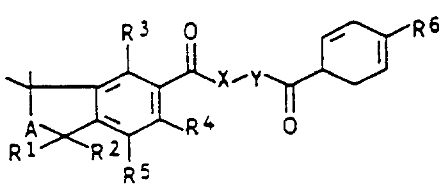

III

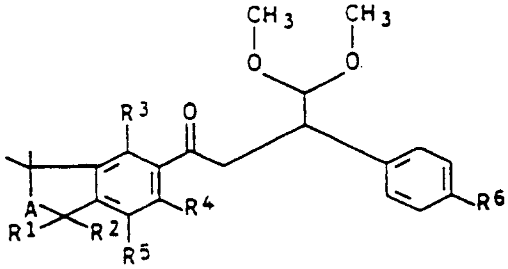

IV

where A and $R^1$ to $R^6$ have the meanings stated in claims 1 to 10 and
X and Y in formula III are each $-CH_2-$ or -NH-, with a dehydrating agent, with or without a sulfide or hydrogen disulfide, ammonia or an ammonium salt, a primary amine or hydrazine to give the corresponding furan, thiophene, pyrrole, pyridazine, oxazole or 1,3,4-oxadiazole;
c) Reaction of a carboxylic acid derivative of the formula V or VI

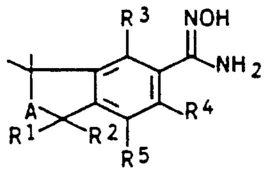

V

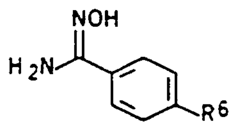

VI

where A and $R^1$ - $R^5$ in formula V and $R^6$ in formula VI have the meanings stated in claims 1 to 10, with another carboxylic acid derivative of the formula VII or VIII

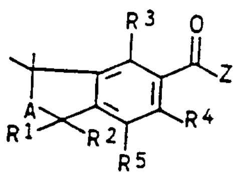

VII

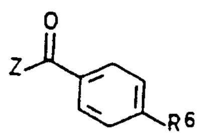

VIII

where A and $R^1$ - $R^5$ in formula VII and $R^6$ in formula VIII have the meanings stated in claims 1 to 10, and Z is chlorine, $C_1$-$C_4$-alkoxy or hydroxyl,
under the conditions of a cyclization reaction, usually of the dehydrating type;
d) 1,3-Dipolar cycloaddition of a nitrile oxide of the formula IX

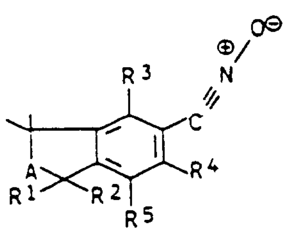

IX

where A and $R^1$ - $R^5$ have the meanings stated in claims 1 to 10, with a monoarylacetylene of the formula X

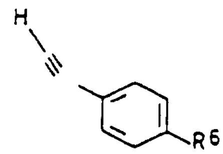

X

where $R^6$ has the meanings stated in claim 1, to give an isoxazole of the formula I.

**15.** A drug for topical administration which contains, in addition to conventional pharmaceutical auxiliaries, from 0.0001 to 1% by weight of a compound of the formula I or Ia as claimed in claim 1 or 2 or 3 or 4 or 5 or 6 or 7 or 8 or 9 or 10 or 11 or 12 as an active compound.

**16.** A drug for systemic administration which contains, in addition to conventional pharmaceutical auxiliaries, from 0.1 to 50 mg, per single dose, of a compound of the formula I or Ia as claimed in claim 1 or 2 or 3 or 4 or 5 or 6 or 7 or 8 or 9 or 10 or 11 or 12 as an active compound.

**17.** A drug as claimed in claim 15 for the treatment of acne or psoriasis or of other dermatological disorders accompanied by pathological changes of hornification and for the treatment of eczemas, warts or vitiligo.

**18.** A drug as claimed in claim 15 for the treatment of skin injuries caused by UV light or iatrogenic skin injuries.

**19.** A drug as claimed in claim 15 or 16 for the treatment of precancerous stages and tumors.

**20.** A drug as claimed in claim 15 or 16 for the treatment of rheumatic and arthritic disorders.

**21.** A drug as claimed in claim 15 or 16 for the treatment of dry eyes and other corneopathies.

**22.** A cosmetic for topical administration, which contains, in addition to conventional cosmetic auxiliaries, from 0.0001 to 1 % by weight of a compound of the formula I or Ia as claimed in claim 1 or 2 or 3 or 4 or 5 or 6 or 7 or 8 or 9 or 10 or 11 or 12 as an active compound.

**23.** A cosmetic as claimed in claim 15 for the treatment of acne or psoriasis or of other dermatological disorders accompanied by pathological changes of hornification and for the treatment of eczemas, warts or vitiligo.

**24.** A cosmetic as claimed in claim 15 for the treatment of skin injuries caused by UV light or iatrogenic skin injuries.

**Revendications**

**1.** Composés de la formule générale I

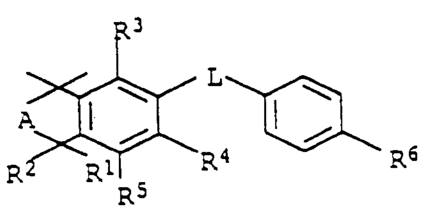

I,

dans laquelle

| | |
|---|---|
| $R^1$ et $R^2$ | représentent hydrogène ou méthyle |
| A | représente un reste éthylène ou méthylène éventuellement substitué avec un groupe méthyle, hydroxyle ou oxo, |
| L | représente un hétérocycle à 5 ou 6 chaînons, saturé ou insaturé avec 1 à 3 hétéroato- |

mes du groupe N, O et S, le 2ème et le 3ème hétéroatome étant un atome d'azote, qui peut être substitué par un groupe hydroxyle-, mercapto-, alkyle en C1-C6 ou alcanoyle en C1-C4 et, dans le cas du pyrazol-1,3-diyle ainsi que du Δ2-pyrazoline-1,3-diyle, peut être substitué en position 3 aussi par alcoxy en C1-C4-carbonyle,

$R^3$ est hydrogène, un groupe hydroxy ou un groupe alcoxy en C1-C6,

$R^4$ est mis pour hydrogène, un reste alkyle en C1-C4, un atome d'halogène ou un groupe méthoxy,

$R^5$ est hydrogène ou un groupe méthoxy ou un groupe t-butyle et

$R^6$ représente hydrogène, un groupe méthyle ou nitrile, un groupe cétal en C2-C10 ou les restes $-CHR^7-OR^8$, $-CHR^7-NR^9R^{10}$, $-COR^{11}$

$-SR^{12}$ ,

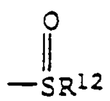 oder 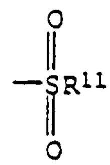

où

$R^7$ est hydrogène ou un groupe alkyle en C1-C4,

$R^8$, hydrogène, un groupe alkyle en C1-C4, un groupe alcanoyle en C1-C20 ou un groupe benzoyle éventuellement substitué par un groupe méthoxy, nitro ou méthyle ou par chlore,

$R^9$ et $R^{10}$ représentent des atomes d'hydrogène, des groupes alkyle en C1-C4, alcanoyle en C1-C6 ou benzoyle éventuellement substitué comme pour $R^8$ ou avec l'atome d'azote auquel ils sont liés, un reste hétérocyclique saturé à 5 ou 6 chaînons, qui peut contenir oxygène comme 2ème hétéroatome,

$R^{11}$ Rreprésente un atome d'hydrogène, un groupe alkyle en C1-C4 ou les restes $-OR^{13}$ ou $-NR^{14}R^{15}$ avec $R^{13}$ représentant hydrogène, un groupe alkyle en C1-C8 éventuellement substitué par des groupes hydroxyle, un groupe aryle ou aralkyle, éventuellement substitué dans la partie aryle, éventuellement substitué par un atome de chlore ou de brome ou un groupe méthyle, méthoxy ou nitro, et avec $R^{14}$ et $R^{15}$ représentant des atomes d'hydrogène, des groupes alkyle en C1-C6 éventuellement substitués par un groupe hydroxy, des groupes aralkyle ou aryle éventuellement substitués comme pour $R^{13}$, ou $R^{14}$ et $R^{15}$ ensemble avec l'atome d'azote auquel ils sont liés, représentant un reste hétérocyclique défini comme plus haut pour $R^9$ et $R^{10}$ et

$R^{12}$ représente un groupe alkyle en C1-C4.

**2.** Composés de la formule générale I selon la revendication 1, $R^6$ représentant un groupe nitrile, un groupe cétal en C2-C10 ou un des restes $-CHR^7-OR^8$, $-CHR^7-NR^9R^{10}$, $-COR^{11}$, $-SR^{12}$, $-S(O)R^{12}$ ou $-S(O)_2R^{11}$.

**3.** Composés de la formule générale I selon la revendication 1, $R^6$ représentant un groupe cétal en C2-C10 ou un des restes $-COR^{11}$, $-S(O)R^{12}$ ou $-SO_2R^{11}$.

**4.** Composés de la formule générale I selon la revendication 1, $R^6$ représentant un des restes $-COR^{11}$ ou $SO_2R^{11}$.

**5.** Composés de la formule générale I selon la revendication 1, L représentant Δ2-pyrazoline-5-one-1,3-diyle.

**6.** Composés de la formule générale I selon la revendication 1, L représentant pyrazole-1,3-diyle ou Δ2-pyrazoline-1,3-diyle et ces noyaux pouvant être substitués chacun en position -5 par hydroxyle, alkyle en C1-C3 ou alcoxy en C1-C4-carbonyle.

**7.** Composés de la formule générale I selon la revendication 1, L représentant un noyau furane, thiophène ou pyrrole et $R^6$ un des restes $-COR^{11}$ et $-SO_2R^{11}$.

**8.** Composés de la formule générale I selon la revendication 1, L représentant Δ2-pyrazoline-5-one-1,3-diyle et R6 un des restes $-COR^{11}$ et $-SO_2R^{11}$.

**9.** Composés de la formule générale I selon la revendication 1, L représentant pyrazol-1,3-diyle ou Δ2-pyrazoline-1,3-diyle et ces noyaux pouvant être chacun substitués en position -5 par hydroxyle, alkyle en C1-C3 ou alcoxy en C1-C4 et $R^6$ représentant un des restes $-COR^{11}$ et $-SO_2R^{11}$.

**10.** Composés de la formule générale I selon la revendication 1, L représentant furane, thiophène ou pyrrole-2,4- ou -2,5-diyle et $R^6$ les groupes carboxyle.

**11.** 2-[(4-méthylsulfonyl)phényl]-5-(5,6,7,8-tétrahydro-5,5,8,8-tétraméthylnaphtalène-2-yl)pyrazoline-3-one.

**12.** Composés de la formule générale Ia

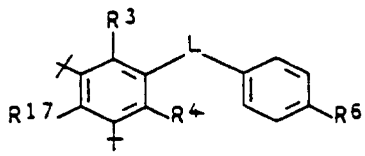

Ia

dans laquelle
$R^3$, $R^4$, $R^6$ et L ont les significations indiquées dans la revendication 1, et $R^{17}$ est H ou OH, ainsi que leurs sels acceptables physiologiquement.

**13.** 2-[(4-méthylsulfonyl)phényl]-5-[(3,5-di-tert-butyl-4-hydroxy)phényl]-pyrazoline-3-one.

**14.** Procédé de préparation de composés de la formule générale I selon la revendication 1, par les réactions suivantes connues en soi :

a) réaction d'un composé 1,3-dicéto de formule II

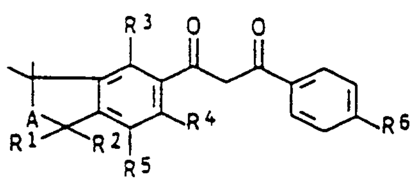

II,

où A et $R^1$ à $R^6$ ont les significations données dans les revendications 1 à 10 avec hydrazine, hydroxylamine ou urée pour aboutir aux pyrazole, isoxazole ou 2-hydroxypyrimidine correspondants ;

b) réaction d'un composé 1,4-dicéto ou son cétal de formule III ou IV

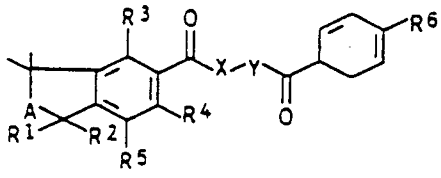

III,

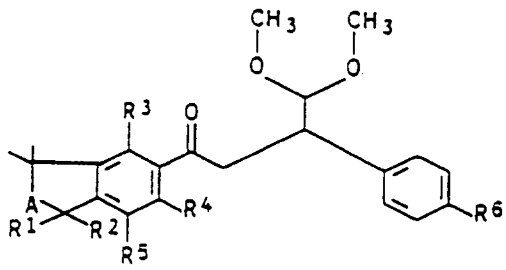

IV,

où

A et $R^1$ à $R^6$ ont les significations données dans les revendications 1 à 10 et

X et Y en formule III sont mis pour $-CH_2$ ou -NH- avec des agents déshydratants, sans ou avec des sulfures ou acide sulfhydrique, ammoniac ou sels d'ammonium, aminés primaires ou hydrazine, pour aboutir aux furane, thiophène, pyrrole, pyridazine, oxazole ou 1,3,4-oxadiazole correspondant ;

c) réaction d'un dérivé d'acide carboxylique de formule V ou VI

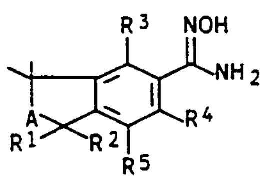

V,

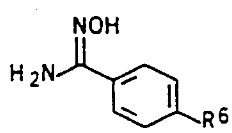

VI,

A et $R^1$-$R^5$ en formule V et $R^6$ en formule VI ont les significations données dans les revendications 1 à 10,

avec un autre dérivé d'acide carboxylique de formule VII ou VIII

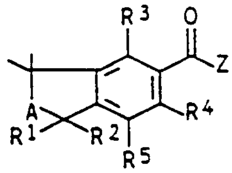

VII,

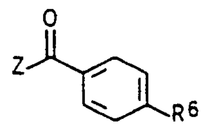

VIII,

où

A et $R^1$ à $R^5$ de formule VII et $R^6$ de formule VIII ont les significations données dans les revendications 1 à 10 et

Z est mis pour chlore, un groupe alcoxy en C1-C4 ou hydroxy

en cyclisant, dans les conditions de réaction déshydratisantes de manière usuelle ;

d) cycloaddition 1,3-dipolaire d'un nitriloxyde de formule IX

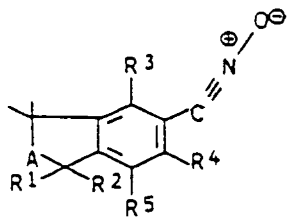

IX,

où

A et $R^1$ à $R^5$ ont les significations données dans les revendications 1 à 10 sur un monoarylacétylène de formule X

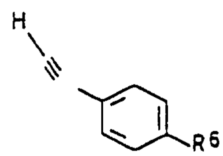

X,

où

$R^6$ a la signification indiquée dans la revendication 1 pour obtenir de l'isoxazolène de formule I.

**15.** Médicament pour utilisation topique qui contient comme principe actif à côté des auxiliaires galéniques usuels, 0,0001 à 1 % en poids d'un composé des formules générales I et Ia selon l'une des revendications 1 à 12.

**16.** Médicament pour utilisation systémique qui contient comme principe actif à côté des auxiliaires galéniques usuels par dose individuelle 0,1 à 50 mg d'un composé des formules générales I et Ia selon l'une des revendications 1 à 12.

**17.** Médicament selon la revendication 15 pour le traitement d'acné ou de psoriasis ou autres maladies dermatologiques s'accompagnant de modifications de l'épiderme corné évoluant pathologiquement, ainsi que les eczémas, verrues et vitiligo.

**18.** Médicament selon la revendication 15 pour le traitement de dommages de la peau dûs à la lumière UV ou iatrogènes.

**19.** Médicament selon la revendication 15 ou 16 pour le traitement de précancéroses et de tumeurs.

**20.** Médicament selon la revendication 15 ou 16 pour le traitement des maladies rhumatismales et arthritiques.

**21.** Médicament selon la revendication 15 ou 16 pour le traitement des maladies secs et autres cornéopathies.

**22.** Cosmétique pour utilisation topique qui contient comme principe actif à côté des auxiliaires cosmétiques usuels, 0,0001 à 1 % en poids d'un composé des formules générales I et Ia selon l'une des revendications 1 à 12.

**23.** Cosmétique selon la revendication 15 pour le traitement d'acné ou de psoriasis ou autres maladies dermatologiques s'accompagnant de modifications de l'épiderme corné évoluant pathologiquement, ainsi que les eczémas, verrues et vitiligo.

**24.** Cosmétique selon la revendication 15 pour le traitement de dommages de la peau dûs à la lumière UV ou iatrogènes.